# EUROPEAN PATENT APPLICATION

(11) **EP 3 831 389 A1**
(43) Date of publication of application: **09.06.2021**
(21) Application number: 20215322.7
(22) Date of filing: 23.11.2016
(51) Int. Cl.: A61K 31/69, A61K 9/00, A61K 45/06, A61K 47/06, A61K 9/06, A61P 17/00, A61P 17/06, A61P 29/00

(54) **TOPICAL PHARMACEUTICAL FORMULATIONS COMPRISING CRISABOROLE FOR TREATING INFLAMMATORY-RELATED CONDITIONS**

(30) Priority: 30.11.2015 US 201562260716 P; 11.11.2016 US 201662420987 P
(62) Divisional of application: 16805522.6
(71) Applicant: Anacor Pharmaceuticals, Inc, New York, NY 10017 (US)
(72) Inventor: CORONADO, Dina Jean, Danville, CA California 94526 (US); IMBERT, Delphine Caroline, Cupertino, CA California 95014 (US); LEE, Charles Edward, Union City, CA California 94587 (US); MERCHANT, Tejal, Cupertino, CA California 95014 (US); YEP, Sylvia Zarela, Milpitas, CA California 95035 (US)
(74) Representative: Pfizer

(57) **Abstract**

Topical pharmaceutical formulations, and methods of treating inflammatory conditions with these formulations, are disclosed.

## Description

### BACKGROUND FOR THE INVENTION

Topical pharmaceutical formulations which are useful in the treatment of inflammatory-related conditions, such as atopic dermatitis and/or psoriasis, are known in the art. Topical pharmaceutical formulations which more quickly reduce the condition symptoms and/or resolve the underlying causes of the condition would be a significant advance in the art.

5-(4-cyanophenoxy)-1,3-dihydro-1-hydroxy-2,1 -benzoxaborole, 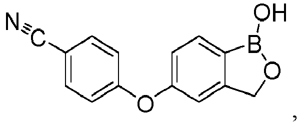 is a non-steroidal PDE4 inhibitor useful in the treatment of inflammatory skin deseases, including mild to moderate atopic dermatitis and psoriasis. Crisaborole (tradename) is 2% 5-(4-cyanophenoxy)-1,3-dihydro-1-hydroxy-2,1-benzoxaborole and is the first topical ointment PDE4 inhibitor for mild to moderate atopic dermatitis (AD) for patients two years of age and older and is recommended for twice daily application to the affected areas for about 28 days and up to an additional 48 weeks.

U.S. Patent Nos. 8,039,451, 8,168,614, 8,501,712 cover the compound and various method of treatments thereof. All references cited herein are incorporated in its entirety and for all purposes.

Formulation development of Crisaborole began with ointment and cream formulations for Phase I and Phase 2 clinical studies. It was determined that an ointment formulation was preferable for the treatment of inflammatory skin diseases, in part due to the beneficial emollient properties of an ointment. Early formulations were comprised of a partial suspension of Crisaborole, but chemical and physical stability issues became problematic requiring a different approach.

The present invention is directed to pharmaceutical compositions containing crisaborole, combinations of crisaborole and other active agents, and methods of using thereof.

### SUMMARY OF THE INVENTION

In a first aspect, the invention provides a topical pharmaceutical formulation comprising:
a) an active agent which treats an inflammatory-related condition, or a pharmaceutically acceptable salt, or a hydrate or a solvate thereof;
b) from about 5% (w/w) to about 15% (w/w) propylene glycol; and
c) petrolatum.

In a second aspect, the invention provides a topical pharmaceutical formulation comprising:
a) from about 5% (w/w) to about 15% (w/w) propylene glycol;
b) petrolatum;
c) an antioxidant;
d) a stabilizer;
e) an emulsifying agent; and
f) a stiffening agent,
wherein the topical pharmaceutical formulation comprises the active agent, crisaborole.

The invention provides additional topical pharmaceutical formulations, as well as methods for their use and production, and combinations thereof.

The present invention also relates to crystalline forms or a non-crystalline form of 5-(4-cyanophenoxy)-1,3-dihydro-1-hydroxy-2,1-benzoxaborole. The present invention also relates to pharmaceutical compositions comprising a crystalline or non-crystalline form, and to methods for preparing such forms. The invention further relates to the use of a crystalline or non-crystalline form in the topical treatment of various diseases.

The invention also contemplates combinations of active ingredients for the treatment of atopic dermatitis.

### DESCRIPTION OF FIGURES

Figure 1: The equation to calculate the volume of material that physically separated.
Figure 2: Powder x-ray spectrum of crisaborole Form 1
Figure 3: Powder X-ray spectrum of crisaborole Forms 1 (black), 2 (red) & 3 (blue).
Figure 4: Powder x-ray spectrum of crisaborole drug product placebo lot overlaid with Form 1.

### DETAILED DESCRIPTION OF THE INVENTION

### I. Definitions and Abbreviations

As used herein, the singular forms "a," "an", and "the" include plural references unless the context clearly dictates otherwise. For example, reference to "an active agent" includes a single active agent as well as two or more different active agents in combination. It is to be understood that present teaching is not limited to the specific dosage forms, carriers, or the like, disclosed herein and as such may vary.

The abbreviations used herein generally have their conventional meaning within the chemical and biological arts.

### II. Introduction

The present invention relates to topical pharmaceutical formulations. These formulations can be useful in the treatment of inflammatory-related conditions. In one aspect, the formulation contains an active agent. In another aspect, the formulation does not contain an active agent. These formulations are useful in the treatment and/or prevention of atopic dermatitis and/or psoriasis.

### IIa. Topical pharmaceutical formulations

In a first aspect, the invention comprises: a) an active agent, or a pharmaceutically acceptable salt, or a hydrate or a solvate thereof; b) from about 5% (w/w) to about 15% (w/w) of a solvent; and c) a base. In an exemplary embodiment, the topical pharmaceutical formulation further comprises up to about 0.5% (w/w) water. In an exemplary embodiment, the topical pharmaceutical formulation further comprises up to about 0.1% (w/w) water. In an exemplary embodiment, the topical pharmaceutical formulation further comprises up to about 0.01% (w/w) water.

In an exemplary embodiment, all of the components of pharmaceutical formulations are pharmaceutically acceptable.

### II. a. i. Active Agent

In an exemplary embodiment, the topical pharmaceutical formulation comprises an active pharmaceutical ingredient ("active agent"). In an exemplary embodiment, the active agent is an anti-inflammatory agent. In an exemplary embodiment, the active agent is an anti-pruritic agent. In an exemplary embodiment, the active agent treats atopic dermatitis. In an exemplary embodiment, the active agent treats psoriasis. In an exemplary embodiment, the active agent is a compound described herein. In an exemplary embodiment, the active agent is a benzoxaborole.

In an exemplary embodiment, the active agent is disclosed in: PCT/US07/062350; 11/676,120 (now 8,168,614); 60/823,888; 60/774,532; PCT/US09/036250; 12/399,015 (now 8,039,450); 61/148,731; 61/143,700; 61/110,903; 61/105,990; 61/094,406; 61/052,637; 61/034,371; PCT/US11/022780; 13/015,487 (now 8,716,478); 61/298,860; 61/354,187; 61/368,211; PCT/US14/056800; and 61/881,343, the content of each of which is herein incorporated by reference for all purposes. In an exemplary embodiment, the active agent is 5-(3,4-dicyanophenoxy)-1,3-dihydro-1-hydroxy-2,1-benzoxaborole, or a pharmaceutically acceptable salt, hydrate, or solvate thereof. In an exemplary embodiment, the active agent is crisaborole, also known as 5-(4-cyanophenoxy)-1,3-dihydro-1-hydroxy-2,1-benzoxaborole, or a pharmaceutically acceptable salt, hydrate, or solvate thereof. In an exemplary embodiment, the active agent is 5-(3-cyanophenoxy)-1,3-dihydro-1-hydroxy-2,1-benzoxaborole, or a pharmaceutically acceptable salt, hydrate, or solvate thereof. In an exemplary embodiment, the active agent is 5-(2-cyanophenoxy)-1,3-dihydro-1-hydroxy-2,1-benzoxaborole, or a pharmaceutically acceptable salt, hydrate, or solvate thereof.

In an exemplary embodiment, the active ingredient is a steroid. In an exemplary embodiment, the active ingredient is pimecrolimus or tazarotene or tacrolimus or triamcinolone or calcitriol or calcipotriene or betamethasone or clobatsol or halobetasol or diflorasone or mometasone.

In an exemplary embodiment, the active agent is present in a concentration of about 0.1% to about 3.0% (w/w). In an exemplary embodiment, the active agent is present in a concentration of about 0.1% to about 2.0% (w/w). In an exemplary embodiment, the active agent is present in a concentration of about 0.1% to about 1.0% (w/w). In an exemplary embodiment, the active agent is present in a concentration of about 1.0% to about 2.0% (w/w). In an exemplary embodiment, the active agent is present in a concentration of about 1.5% to about 2.0% (w/w). In an exemplary embodiment, the active agent is present in a concentration of about 1.5% to about 2.5% (w/w). In an exemplary embodiment, the active agent is present in a concentration of about 1.0% to about 3.0% (w/w). In an exemplary embodiment, the active agent is present in a concentration of about 2.0% (w/w). In an exemplary embodiment, the active agent is present in a concentration of 2.0% (w/w).

In an exemplary embodiment, the invention provides an active agent described herein, or a salt, hydrate or solvate thereof, or a combination thereof. An exemplary embodiment is a combination of crisaborole as one active agent and a second active agent useful for the treatment of atopic dermatitis or psoriasis. The combination may be comprised of an admixture or co-formulation of the two active ingredients. Alternatively, the combination may be packaged in a dispenser wherein one active agent is in one chamber and another active ingredient is in a second chamber, but upon dispensing the two active agents are simultaneously delivered together such that administration of the combination may occur in one application. Alternatively, the active agents may individually be administered with the other active agent, wherein the second active agent may be administered either orally or topically.

Examples of second active agents that are contemplated in combination with crisaborole include but are not limited to:
Topical corticosteroids such as Fluocinonide, Desoximetasone, Mometasone, Triamcinolone, Betamethasone, Alclometasone, Desonide, Hydrocortisone and Mapracorat;
Topical Calcineurin inhibitors such as Tacrolimus, pimecrolimus and cyclosporine;
Topical formulations of PDE4 inhibitors such as apremilast, E-6005, OPA-15406, LEO 29102, DRM02, and Roflumilast;
Topical formulations of JAK kinase inhibitors such as Tofacitinib, JTE-052, Baricitinib, and Upadacitinib;
Topical Non-steroidal anti-inflammatories such as WBI-1001, and MRX-6;
Topcial ROR agents such as GSK2981278;
Injectable Anti- IL4, IL-31, IL-22, IL-33, IL-12, IL-23, IL-17, IgE, IL-4 treatments such as Dupilumab, Lebrikizumab, Nemolizumab, Tralokinumab, Etanercept, Adalimumab, Infliximab, Ustekinumab, Secukinumab, Omazumilab, CIM-331;
Vitamin D analogs such as calcipotriene ;
Oral Retinoic Acid derivatives such as alitretinoin;
Oral Liver X Receptor (LXR) selective agonists such as VTP-38543 ;
Oral H4 receptor antagonists such as ZPL-389 ;
Oral NK1 receptor antagonists such as Aprepitant and Tradipitant;
Oral CRTH2 receptor antagonists such as Fevipiprant and OC-459 ;
Oral Chymase inhibitors such as SUN 13834;
Oral GATA-3 inhibitors such as SB-011;
Oral ROR inverse agonists such as VTP-43742, ARN6039, TAK-828 and JTE-451;
Oral JAK inhibitors; including inhibitors of JAK1, JAK2, JAK3 and TYK2 such as PF-04965842, PF-06651600, and PF-06700841;
Oral PDE agents such as apremilast, roflumilast, and ibudilast;
Oral IRAK4 inhibitors such as PF-06650833;
Injectable aTNF inhibitors such as infliximab, adalimumab, golimumab, and certolizumab pegol;
Injectable galectin-3 inhibitor such as GR-MD-02

In an exemplary embodiment, the invention provides an active agent described herein, or a salt, hydrate or solvate thereof. In an exemplary embodiment, the invention provides an active agent described herein, or a salt thereof. In an exemplary embodiment, the salt is a pharmaceutically acceptable salt. In an exemplary embodiment, the invention provides an active agent described herein, or a hydrate thereof. In an exemplary embodiment, the invention provides an active agent described herein, or a solvate thereof. In an exemplary embodiment, the invention provides an active agent described herein, or a prodrug thereof. In an exemplary embodiment, the invention provides an active agent described herein. In an exemplary embodiment, the invention provides a pharmaceutically acceptable salt of an active agent described herein. In an exemplary embodiment, the invention provides a hydrate of an active agent described herein. In an exemplary embodiment, the invention provides a solvate of an active agent described herein. In an exemplary embodiment, the invention provides a prodrug of an active agent described herein.

### II. a. ii. Solvent

In an exemplary embodiment, the topical pharmaceutical formulation comprises a solvent. In an exemplary embodiment, the solvent is an alkylene glycol. In an exemplary embodiment, the solvent is propylene glycol. In an exemplary embodiment, the solvent is propylene glycol USP. In an exemplary embodiment, the solvent is butylene glycol.

In an exemplary embodiment, the solvent is present in a concentration of about 5.0% (w/w) to about 15.0% (w/w). In an exemplary embodiment, the solvent is present in a concentration of about 6.0% (w/w) to about 10.0% (w/w). In an exemplary embodiment, the solvent is present in a concentration of about 6.5% (w/w) to about 11.5% (w/w). In an exemplary embodiment, the solvent is present in a concentration of about 7.0% (w/w) to about 11.0% (w/w). In an exemplary embodiment, the solvent is present in a concentration of about 7.5% (w/w) to about 10.5% (w/w). In an exemplary embodiment, the solvent is present in a concentration of about 7.5% (w/w) to about 9.5% (w/w). In an exemplary embodiment, the solvent is present in a concentration of about 8.5% (w/w) to about 9.5% (w/w). In an exemplary embodiment, the solvent is present in a concentration of about 8.0% (w/w) to about 10.0% (w/w). In an exemplary embodiment, the solvent is present in a concentration of about 9.0% (w/w). In an exemplary embodiment, the solvent is present in a concentration of 9.0% (w/w).

### II. a. iii. Base

In an exemplary embodiment, the topical pharmaceutical formulation comprises an ointment base. In an exemplary embodiment, the base is an ointment base. In an exemplary embodiment, the ointment base is white petrolatum. In an exemplary embodiment, the ointment base is white petrolatum USP. In an exemplary embodiment, the ointment base is mineral jelly or petroleum jelly or yellow petrolatum or yellow soft paraffin or yellow petroleum Jelly or white petrolatum jelly or white soft paraffin. In an exemplary embodiment, the base is mineral oil or light mineral oil or paraffin or lanolin alcohol.

The amount of base in the topical pharmaceutical formulations will be dependent on the amounts of the other components. More base may be added to compensate for smaller amounts of other components in the desired topical pharmaceutical formulation. In an exemplary embodiment, the base is present in a quantum satis, q.s., concentration. In an exemplary embodiment, the base is present in a concentration of from about 65% (w/w) to about 90% (w/w). In an exemplary embodiment, the base is present in a concentration of from about 65% (w/w) to about 85% (w/w). In an exemplary embodiment, the base is present in a concentration of from about 67.955% (w/w) to about 89.8999% (w/w). In an exemplary embodiment, the base is present in a concentration of from about 50% (w/w) to about 60% (w/w). In an exemplary embodiment, the base is present in a concentration of from about 60% (w/w) to about 70% (w/w). In an exemplary embodiment, the base is present in a concentration of from about 70% (w/w) to about 80% (w/w). In an exemplary embodiment, the base is present in a concentration of from about 72% (w/w) to about 82% (w/w). In an exemplary embodiment, the base is present in a concentration of from about 74% (w/w) to about 81% (w/w). In an exemplary embodiment, the base is present in a concentration of from about 78% (w/w) to about 82% (w/w). In an exemplary embodiment, the base is present in a concentration of from about 75% (w/w) to about 80% (w/w). In an exemplary embodiment, the base is present in a concentration of from about 75% (w/w) to about 79% (w/w). In an exemplary embodiment, the base is present in a concentration of from about 76% (w/w) to about 79% (w/w). In an exemplary embodiment, the base is present in a concentration of from about 76% (w/w) to about 77% (w/w). In an exemplary embodiment, the base is present in a concentration of from about 76.8% (w/w) to about 77% (w/w). In an exemplary embodiment, the base is present in a concentration of from about 78% (w/w) to about 79% (w/w). In an exemplary embodiment, the base is present in a concentration of from about 76.8% (w/w) to about 76.9% (w/w). In an exemplary embodiment, the base is present in a concentration of from about 76.89% (w/w) to about 76.9% (w/w). In an exemplary embodiment, the base is present in a concentration of about 76.80% (w/w). In an exemplary embodiment, the base is present in a concentration of about 76.855% (w/w). In an exemplary embodiment, the base is present in a concentration of about 76.8965% (w/w). In an exemplary embodiment, the base is present in a concentration of about 76.8976% (w/w). In an exemplary embodiment, the base is present in a concentration of about 76.981% (w/w). In an exemplary embodiment, the base is present in a concentration of about 76.90% (w/w). In an exemplary embodiment, the base is present in a concentration of from about 78.89% (w/w) to about 78.9% (w/w). In an exemplary embodiment, the base is present in a concentration of about 78.80% (w/w). In an exemplary embodiment, the base is present in a concentration of about 78.855% (w/w). In an exemplary embodiment, the base is present in a concentration of about 78.8965% (w/w). In an exemplary embodiment, the base is present in a concentration of about 78.8976% (w/w). In an exemplary embodiment, the base is present in a concentration of about 78.981% (w/w). In an exemplary embodiment, the base is present in a concentration of about 78.90% (w/w).

### Optional components for the topical pharmaceutical formulation

### II. a. iv. Antioxidant

In an exemplary embodiment, the topical pharmaceutical formulation further comprises an antioxidant. In an exemplary embodiment, the antioxidant is selected from the group consisting of butylated hydroxytoluene, ascorbic acid, ascorbic palmitate, butylated hydroxyanisole, 2,4,5-trihydroxybutyrophenone, 4-hydroxymethyl-2,6-di-tertbutylphenol, erythorbic acid, gum guaiac, propyl gallate, thiodipropionic acid, dilauryl thiodipropionate, tert-butylhydroquinone and a tocopherol, or a pharmaceutically acceptable salt or ester thereof, or a combination thereof. In an exemplary embodiment, the antioxidant is butylated hydroxytoluene. In an exemplary embodiment, the antioxidant is butylated hydroxytoluene NF.

In an exemplary embodiment, the antioxidant is present in a concentration of about 0.01% (w/w) to about 1% (w/w). In an exemplary embodiment, the antioxidant is present in a concentration of about 0.01% (w/w) to about 0.5% (w/w). In an exemplary embodiment, the antioxidant is present in a concentration of about 0.05% (w/w) to about 0.5% (w/w). In an exemplary embodiment, the antioxidant is present in a concentration of about 0.05% (w/w) to about 0.4% (w/w). In an exemplary embodiment, the antioxidant is present in a concentration of about 0.05% (w/w) to about 0.3% (w/w). In an exemplary embodiment, the antioxidant is present in a concentration of about 0.07% (w/w) to about 0.2% (w/w). In an exemplary embodiment, the antioxidant is present in a concentration of about 0.05% (w/w) to about 0.15% (w/w). In an exemplary embodiment, the antioxidant is present in a concentration of about 0.1% (w/w). In an exemplary embodiment, the antioxidant is present in a concentration of 0.1% (w/w).

### II. a. v. Stabilizer

In an exemplary embodiment, the topical pharmaceutical formulation further comprises a stabilizer. In an exemplary embodiment, the stabilizer is ethylenediaminetetraacetic acid, or a pharmaceutically acceptable salt thereof. In an exemplary embodiment, the stabilizer is a pharmaceutically acceptable salt of ethylenediaminetetraacetic acid, and this salt is a sodium salt or a potassium salt or a calcium salt, or a combination thereof. In an exemplary embodiment, the stabilizer is a pharmaceutically acceptable salt of ethylenediaminetetraacetic acid, and this salt is a sodium salt or a calcium salt, or a combination thereof. In an exemplary embodiment, the stabilizer is edetate calcium disodium. In an exemplary embodiment, the stabilizer is edetate calcium disodium USP.

In an exemplary embodiment, the stabilizer is present in a concentration of about 0.000010% (w/w) to about 0.0450% (w/w). In an exemplary embodiment, the stabilizer is present in a concentration of about 0.0010% (w/w) to about 0.0450% (w/w). In an exemplary embodiment, the stabilizer is present in a concentration of about 0.0010% (w/w) to about 0.0400% (w/w). In an exemplary embodiment, the stabilizer is present in a concentration of about 0.0010% (w/w) to about 0.0350% (w/w). In an exemplary embodiment, the stabilizer is present in a concentration of about 0.0010% (w/w) to about 0.0300% (w/w). In an exemplary embodiment, the stabilizer is present in a concentration of about 0.0010% (w/w) to about 0.0250% (w/w). In an exemplary embodiment, the stabilizer is present in a concentration of about 0.0010% (w/w) to about 0.0200% (w/w). In an exemplary embodiment, the stabilizer is present in a concentration of about 0.0010% (w/w) to about 0.0150% (w/w). In an exemplary embodiment, the stabilizer is present in a concentration of about 0.00010% (w/w) to about 0.0100% (w/w). In an exemplary embodiment, the stabilizer is present in a concentration of about 0.0010% (w/w) to about 0.0090% (w/w). In an exemplary embodiment, the stabilizer is present in a concentration of about 0.000010% (w/w) to about 0.0100% (w/w). In an exemplary embodiment, the stabilizer is present in a concentration of about 0.0010% (w/w) to about 0.0100% (w/w). In an exemplary embodiment, the stabilizer is present in a concentration of about 0.0020% (w/w) to about 0.0100% (w/w). In an exemplary embodiment, the stabilizer is present in a concentration of about 0.0024% (w/w) to about 0.0100% (w/w). In an exemplary embodiment, the stabilizer is present in a concentration of about 0.0024% (w/w) to about 0.0090% (w/w). In an exemplary embodiment, the stabilizer is present in a concentration of about 0.0035% (w/w) to about 0.0100% (w/w). In an exemplary embodiment, the stabilizer is present in a concentration of about 0.0035% (w/w) to about 0.0090% (w/w). In an exemplary embodiment, the stabilizer is present in a concentration of about 0.0010% (w/w) to about 0.0080% (w/w). In an exemplary embodiment, the stabilizer is present in a concentration of about 0.0010% (w/w) to about 0.0060% (w/w). In an exemplary embodiment, the stabilizer is present in a concentration of about 0.0010% (w/w) to about 0.0050% (w/w). In an exemplary embodiment, the stabilizer is present in a concentration of about 0.0020% (w/w) to about 0.0060% (w/w). In an exemplary embodiment, the stabilizer is present in a concentration of about 0.0015% (w/w) to about 0.0045% (w/w). In an exemplary embodiment, the stabilizer is present in a concentration of about 0.0025% (w/w) to about 0.0045% (w/w). In an exemplary embodiment, the stabilizer is present in a concentration of about 0.0030% (w/w) to about 0.0040% (w/w). In an exemplary embodiment, the stabilizer is present in a concentration of about 0.0035% (w/w). In an exemplary embodiment, the stabilizer is present in a concentration of 0.0035% (w/w).

### II. a. vi. Emulsifying Agent

In an exemplary embodiment, the topical pharmaceutical formulation further comprises an emulsifying agent. In an exemplary embodiment, the emulsifying agent is a glyceride blend. In an exemplary embodiment, the emulsifying agent is a glyceride blend, wherein the glyceride blend comprises a monoglyceride and a diglyceride. In an exemplary embodiment, the emulsifying agent is a glyceride blend, wherein the glyceride blend comprises a monoglyceride, a diglyceride, and a triglyceride. In an exemplary embodiment, the emulsifying agent is a glyceride blend, wherein the glyceride blend comprises a monoglyceride and a diglyceride, and wherein from about 40% (w/w) to about 55% (w/w) of the glyceride blend is a monoglyceride. In an exemplary embodiment, the emulsifying agent is a glyceride blend, wherein the glyceride blend comprises a monoglyceride, a diglyceride, and a triglyceride, and wherein from about 40% (w/w) to about 55% (w/w) of the glyceride blend is a monoglyceride. In an exemplary embodiment, the emulsifying agent is a glyceride blend, wherein the glyceride blend is Mono- and Di-glyceride NF.

In an exemplary embodiment, the monoglyceride is selected from the group consisting of glyceryl monostearate, glyceryl monopalmitate, glyceryl monooleate, or combinations thereof. In an exemplary embodiment, the monoglyceride is a monoglyceryl ester of a long chain, saturated or unsaturated fatty acid. In an exemplary embodiment, the monoglyceride is an alpha-monoglyceride. In an exemplary embodiment, the diglyceride is a diglyceryl ester of a long chain, saturated or unsaturated fatty acid.

In an exemplary embodiment, the glyceride blend is present in a concentration of about 3.0% (w/w) to about 10.0% (w/w). In an exemplary embodiment, the glyceride blend is present in a concentration of about 5.0% (w/w) to about 10.0% (w/w). In an exemplary embodiment, the glyceride blend is present in a concentration of about 6.0% (w/w) to about 9.0% (w/w). In an exemplary embodiment, the glyceride blend is present in a concentration of about 5.0% (w/w) to about 8.0% (w/w). In an exemplary embodiment, the glyceride blend is present in a concentration of about 6.0% (w/w) to about 8.0% (w/w). In an exemplary embodiment, the glyceride blend is present in a concentration of about 6.5% (w/w) to about 7.5% (w/w). In an exemplary embodiment, the glyceride blend is present in a concentration of about 7.0% (w/w). In an exemplary embodiment, the glyceride blend is present in a concentration of 7.0% (w/w).

### II. a. vii. Stiffening agent

In an exemplary embodiment, the topical pharmaceutical formulation further comprises a stiffening agent. In an exemplary embodiment, the stiffening agent is a wax. In an exemplary embodiment, the stiffening agent is a wax, and the wax is selected from the group consisting of beeswax, paraffin wax, and spermaceti wax. In an exemplary embodiment, the stiffening agent is paraffin wax. In an exemplary embodiment, the stiffening agent is paraffin wax NF.

In an exemplary embodiment, the stiffening agent is present in a concentration of about 2.0% (w/w) to about 6.0% (w/w). In an exemplary embodiment, the stiffening agent is present in a concentration of about 2.0% (w/w) to about 8.0% (w/w). In an exemplary embodiment, the stiffening agent is present in a concentration of about 3.0% (w/w) to about 5.0% (w/w). In an exemplary embodiment, the stiffening agent is present in a concentration of about 4.0% (w/w) to about 6.0% (w/w). In an exemplary embodiment, the stiffening agent is present in a concentration of about 4.0% (w/w) to about 5.0% (w/w). In an exemplary embodiment, the stiffening agent is present in a concentration of about 4.5% (w/w) to about 5.5% (w/w). In an exemplary embodiment, the stiffening agent is present in a concentration of about 5.0% (w/w). In an exemplary embodiment, the stiffening agent is present in a concentration of 5.0% (w/w).

### Specific topical pharmaceutical formulations

### II. a. viii.

In an exemplary embodiment, the topical pharmaceutical formulation comprises:
a) an active agent which treats atopic dermatitis and/or psoriasis, or a pharmaceutically acceptable salt, or a hydrate or a solvate thereof;
b) from about 5% (w/w) to about 15% (w/w) propylene glycol; and
c) petrolatum
wherein the formulation comprises no more than about 0.5% (w/w) water.

In an exemplary embodiment, the topical pharmaceutical formulation comprises:
a) an active agent which treats atopic dermatitis and/or psoriasis, or a pharmaceutically acceptable salt, or a hydrate or a solvate thereof;
b) from about 5% (w/w) to about 15% (w/w) propylene glycol;
c) petrolatum;
d) an antioxidant;
e) a stabilizer;
f) an emulsifying agent; and
g) a stiffening agent
wherein the formulation comprises no more than about 0.5% (w/w) water.

In an exemplary embodiment, the topical pharmaceutical formulation comprises:
a) an active agent which treats atopic dermatitis and/or psoriasis, or a pharmaceutically acceptable salt, or a hydrate or a solvate thereof;
b) from about 8% (w/w) to about 10% (w/w) propylene glycol;
c) petrolatum;
d) an antioxidant;
e) a stabilizer;
f) an emulsifying agent; and
g) a stiffening agent
wherein the formulation comprises no more than about 0.5% (w/w) water.

In an exemplary embodiment, the topical pharmaceutical formulation consists of:
a) an active agent which treats atopic dermatitis and/or psoriasis, or a pharmaceutically acceptable salt, or a hydrate or a solvate thereof;
b) from about 8% (w/w) to about 10% (w/w) propylene glycol;
c) petrolatum;
d) an antioxidant;
e) edetate calcium disodium;
f) an emulsifying agent; and
g) a stiffening agent.

In an exemplary embodiment, the topical pharmaceutical formulation consists of:
a) an active agent which treats atopic dermatitis and/or psoriasis, or a pharmaceutically acceptable salt, or a hydrate or a solvate thereof;
b) from about 8% (w/w) to about 10% (w/w) propylene glycol;
c) petrolatum;
d) an antioxidant;
e) from about 0.0020% (w/w) to about 0.0040% (w/w) edetate calcium disodium;
f) an emulsifying agent; and
g) a stiffening agent.

In an exemplary embodiment, the topical pharmaceutical formulation consists essentially of:
a) an active agent which treats atopic dermatitis and/or psoriasis, or a pharmaceutically acceptable salt, or a hydrate or a solvate thereof;
b) from about 8% (w/w) to about 10% (w/w) propylene glycol;
c) petrolatum;
d) an antioxidant;
e) a stabilizer;
f) a glyceride blend, wherein the glyceride blend comprises one or more monoglycerides and one or more diglycerides; and
g) a stiffening agent
wherein the formulation comprises no more than about 0.5% (w/w) water.

In an exemplary embodiment, the topical pharmaceutical formulation consists essentially of:
a) an active agent which treats atopic dermatitis and/or psoriasis, or a pharmaceutically acceptable salt, or a hydrate or a solvate thereof;
b) from about 8% (w/w) to about 10% (w/w) propylene glycol;
c) petrolatum;
d) an antioxidant;
e) a stabilizer;
f) a glyceride blend, wherein the glyceride blend comprises one or more monoglycerides and one or more diglycerides, wherein the one or more monoglycerides is in a total concentration of between 40% and 55% of the glyceride blend; and
g) a stiffening agent
wherein the formulation comprises no more than about 0.5% (w/w) water.

In an exemplary embodiment, the topical pharmaceutical formulation consists essentially of:
a) an active agent which treats atopic dermatitis and/or psoriasis, or a pharmaceutically acceptable salt, or a hydrate or a solvate thereof;
b) from about 8% (w/w) to about 10% (w/w) propylene glycol;
c) petrolatum;
d) an antioxidant;
e) a stabilizer;
f) from about 5% (w/w) to about 10% (w/w) of a glyceride blend, wherein the glyceride blend comprises one or more monoglycerides and one or more diglycerides, wherein the one or more monoglycerides is in a total concentration of between 40% and 55% of the glyceride blend; and
g) a stiffening agent
wherein the formulation comprises no more than about 0.5% (w/w) water.

In an exemplary embodiment, the topical pharmaceutical formulation consists essentially of:
a) an active agent which treats atopic dermatitis and/or psoriasis, or a pharmaceutically acceptable salt, or a hydrate or a solvate thereof;
b) from about 8% (w/w) to about 10% (w/w) propylene glycol;
c) petrolatum;
d) an antioxidant;
e) a stabilizer;
f) from about 6% (w/w) to about 8% (w/w) of a glyceride blend, wherein the glyceride blend comprises one or more monoglycerides and one or more diglycerides, wherein the one or more monoglycerides is in a total concentration of between 40% and 55% of the glyceride blend; and
g) a stiffening agent
wherein the formulation comprises no more than about 0.5% (w/w) water.

In an exemplary embodiment, the topical pharmaceutical formulation consists essentially of:
a) an active agent which treats atopic dermatitis and/or psoriasis, or a pharmaceutically acceptable salt, or a hydrate or a solvate thereof;
b) from about 8% (w/w) to about 10% (w/w) propylene glycol;
c) petrolatum;
d) an antioxidant;
e) edetate calcium disodium;
f) from about 5% (w/w) to about 10% (w/w) of a glyceride blend, wherein the glyceride blend comprises one or more monoglycerides and one or more diglycerides, wherein the one or more monoglycerides is in a total concentration of between 40% and 55% of the glyceride blend; and
g) a stiffening agent
wherein the formulation comprises no more than about 0.5% (w/w) water.

In an exemplary embodiment, the topical pharmaceutical formulation consists essentially of:
a) an active agent which treats atopic dermatitis and/or psoriasis, or a pharmaceutically acceptable salt, or a hydrate or a solvate thereof;
b) from about 8% (w/w) to about 10% (w/w) propylene glycol;
c) petrolatum;
d) an antioxidant;
e) from about 0.0020% (w/w) to about 0.0040% (w/w) edetate calcium disodium;
f) from about 5% (w/w) to about 10% (w/w) of a glyceride blend, wherein the glyceride blend comprises one or more monoglycerides and one or more diglycerides, wherein the one or more monoglycerides is in a total concentration of between 40% and 55% of the glyceride blend; and
g) a stiffening agent
wherein the formulation comprises no more than about 0.5% (w/w) water.

In an exemplary embodiment, the topical pharmaceutical formulation consists essentially of:
a) 5-(4-cyanophenoxy)-1,3-dihydro-1-hydroxy-2,1-benzoxaborole, or a pharmaceutically acceptable salt, or a hydrate or a solvate thereof;
b) from about 8% (w/w) to about 10% (w/w) propylene glycol;
c) petrolatum;
d) an antioxidant;
e) a stabilizer;
f) from about 5% (w/w) to about 10% (w/w) of a glyceride blend, wherein the glyceride blend comprises one or more monoglycerides and one or more diglycerides, wherein the one or more monoglycerides is in a total concentration of between 40% and 55% of the glyceride blend; and
g) a stiffening agent
wherein the formulation comprises no more than about 0.5% (w/w) water.

In an exemplary embodiment, the topical pharmaceutical formulation consists essentially of:
a) 5-(4-cyanophenoxy)-1,3-dihydro-1-hydroxy-2,1-benzoxaborole, or a pharmaceutically acceptable salt, or a hydrate or a solvate thereof;
b) from about 8% (w/w) to about 10% (w/w) propylene glycol;
c) petrolatum;
d) an antioxidant;
e) edetate calcium disodium;
f) from about 6% (w/w) to about 8% (w/w) of a glyceride blend, wherein the glyceride blend comprises one or more monoglycerides and one or more diglycerides, wherein the one or more monoglycerides is in a total concentration of between 40% and 55% of the glyceride blend; and
g) a stiffening agent
wherein the formulation comprises no more than about 0.5% (w/w) water.

In an exemplary embodiment, the topical pharmaceutical formulation consists essentially of:
a) 5-(4-cyanophenoxy)-1,3-dihydro-1-hydroxy-2,1-benzoxaborole, or a pharmaceutically acceptable salt, or a hydrate or a solvate thereof;
b) from about 8% (w/w) to about 10% (w/w) propylene glycol;
c) petrolatum;
d) an antioxidant;
e) from about 0.0020% (w/w) to about 0.0040% (w/w) edetate calcium disodium;
f) from about 6% (w/w) to about 8% (w/w) of a glyceride blend, wherein the glyceride blend comprises one or more monoglycerides and one or more diglycerides, wherein the one or more monoglycerides is in a total concentration of between 40% and 55% of the glyceride blend; and
g) a stiffening agent
wherein the formulation comprises no more than about 0.5% (w/w) water.

In an exemplary embodiment, the topical pharmaceutical formulation consists essentially of:
a) 5-(4-cyanophenoxy)-1,3-dihydro-1-hydroxy-2,1-benzoxaborole, or a pharmaceutically acceptable salt, or a hydrate or a solvate thereof;
b) from about 5% (w/w) to about 15% (w/w) propylene glycol;
c) petrolatum;
d) butylated hydroxytoluene;
e) edetate calcium disodium;
f) from about 5% (w/w) to about 10% (w/w) of a glyceride blend, wherein the glyceride blend comprises one or more monoglycerides and one or more diglycerides, wherein the one or more monoglycerides is in a total concentration of between 40% and 55% of the glyceride blend; and
g) a stiffening agent
wherein the formulation comprises no more than about 0.5% (w/w) water.

In an exemplary embodiment, the topical pharmaceutical formulation consists essentially of:
a) 5-(4-cyanophenoxy)-1,3-dihydro-1-hydroxy-2,1-benzoxaborole, or a pharmaceutically acceptable salt, or a hydrate or a solvate thereof;
b) from about 5% (w/w) to about 15% (w/w) propylene glycol;
c) petrolatum;
d) butylated hydroxytoluene;
e) edetate calcium disodium;
f) from about 5% (w/w) to about 10% (w/w) of a glyceride blend, wherein the glyceride blend comprises one or more monoglycerides and one or more diglycerides, wherein the one or more monoglycerides is in a total concentration of between 40% and 55% of the glyceride blend; and
g) a stiffening agent
wherein the formulation comprises no more than about 0.5% (w/w) water.

In an exemplary embodiment, the topical pharmaceutical formulation consists essentially of:
a) about 2% (w/w) 5-(4-cyanophenoxy)-1,3-dihydro-1-hydroxy-2,1-benzoxaborole, or a pharmaceutically acceptable salt, or a hydrate or a solvate thereof;
b) about 9% (w/w) propylene glycol;
c) white petrolatum;
d) about 0.1% (w/w) butylated hydroxytoluene;
e) about 0.0035% (w/w) edetate calcium disodium;
f) about 7% (w/w) of a glyceride blend, wherein the glyceride blend comprises one or more monoglycerides and one or more diglycerides, wherein the one or more monoglycerides is in a total concentration of between 40% and 55% of the glyceride blend; and
g) a stiffening agent
wherein the formulation comprises no more than about 0.5% (w/w) water.

In an exemplary embodiment, the topical pharmaceutical formulation consists of:
a) 2% (w/w) 5-(4-cyanophenoxy)-1,3-dihydro-1-hydroxy-2,1-benzoxaborole, or a pharmaceutically acceptable salt, or a hydrate or a solvate thereof;
b) 9% (w/w) propylene glycol;
c) 76.8965% (w/w) white petrolatum;
d) 0.1% (w/w) butylated hydroxytoluene;
e) 0.0035% (w/w) edetate calcium disodium;
f) 7% (w/w) of a glyceride blend, wherein the glyceride blend comprises one or more monoglycerides and one or more diglycerides, wherein the one or more monoglycerides is in a total concentration of between 40% and 55% of the glyceride blend; and
g) 5% (w/w) paraffin wax.

### Topical pharmaceutical formulation without an active agent

### II. a.ix.

In another aspect, the invention comprises a topical pharmaceutical formulation which does not comprise an active agent which is useful in the treatment of inflammatory-related conditions. These topical pharmaceutical formulations which do not contain an active agent are also useful in the treatment of inflammatory conditions such as atopic dermatitis and/or psoriasis. In an exemplary embodiment, these topical pharmaceutical formulation do not contain one or more of the active agents listed herein. In an exemplary embodiment, the topical pharmaceutical formulation without an active agent further comprises up to about 0.5% (w/w) water. In an exemplary embodiment, the topical pharmaceutical formulation without an active agent further comprises up to about 0.1% (w/w) water. In an exemplary embodiment, the topical pharmaceutical formulation without an active agent further comprises up to about 0.01% (w/w) water.

The topical pharmaceutical formulation without an active agent may optionally contain a solvent, a base, an antioxidant, a stabilizer, an emulsifying agent, and a stiffening agent. The identity and concentrations for each of these components in the topical pharmaceutical formulation without an active agent may be found in sections II. a. i.; II. a. ii.; II. a. iii.; II. a. iv.; II. a. v.; II. a. vi.; II. a. vii.; and II. a. viii. of this document.

### Specific topical pharmaceutical formulations without an active agent

### II. a.x.

In an exemplary embodiment, the topical pharmaceutical formulation comprises:
a) from about 5% (w/w) to about 15% (w/w) propylene glycol;
b) petrolatum;
c) an antioxidant;
d) a stabilizer;
e) an emulsifying agent; and
f) a stiffening agent,
wherein the topical pharmaceutical formulation does not comprise an active agent and wherein the formulation comprises no more than about 0.5% (w/w) water.

In an exemplary embodiment, the topical pharmaceutical formulation comprises:
a) about 5% (w/w) of a solvent;
b) about 89.8999% (w/w) of petrolatum;
c) about 0.1% (w/w) of an antioxidant;
d) about 0.0001% (w/w) of a stabilizer;
e) about 3% (w/w) of an emulsifying agent; and
f) about 2% (w/w) of a stiffening agent,
wherein the topical pharmaceutical formulation does not comprise an active agent and wherein the formulation comprises no more than about 0.5% (w/w) water.

In an exemplary embodiment, the topical pharmaceutical formulation comprises:
a) about 15% (w/w) of a solvent;
b) about 67.955% (w/w) of petrolatum;
c) about 1% (w/w) of an antioxidant;
d) about 0.0450% (w/w) of a stabilizer;
e) about 10% (w/w) of an emulsifying agent; and
f) about 6% (w/w) of a stiffening agent,
wherein the topical pharmaceutical formulation does not comprise an active agent and wherein the formulation comprises no more than about 0.5% (w/w) water.

In an exemplary embodiment, the topical pharmaceutical formulation comprises:
a) from about 5% (w/w) to about 15% (w/w) of a solvent;
b) from about 67.955% (w/w) to about 89.8999% (w/w) of petrolatum;
c) from about 0.1% (w/w) to about 1% (w/w) of an antioxidant;
d) from about 0.0001% (w/w) to about 0.0450% (w/w) of a stabilizer;
e) from about 3% (w/w) to about 10% (w/w) of an emulsifying agent; and
f) from about 2% (w/w) to about 6% (w/w) of a stiffening agent,
wherein the topical pharmaceutical formulation does not comprise an active agent and wherein the formulation comprises no more than about 0.5% (w/w) water.

In an exemplary embodiment, the topical pharmaceutical formulation comprises:
a) from about 5% (w/w) to about 15% (w/w) of propylene glycol;
b) from about 67.955% (w/w) to about 89.8999% (w/w) of petrolatum;
c) from about 0.1% (w/w) to about 1% (w/w) of butylated hydroxytoluene;
d) from about 0.0001% (w/w) to about 0.0450% (w/w) of edetate calcium disodium;
e) from about 3% (w/w) to about 10% (w/w) of a glyceride blend; and
f) from about 2% (w/w) to about 6% (w/w) of paraffin wax,
wherein the topical pharmaceutical formulation does not comprise an active agent and wherein the formulation comprises no more than about 0.5% (w/w) water.

In an exemplary embodiment, the topical pharmaceutical formulation comprises:
a) from about 5% (w/w) to about 15% (w/w) of propylene glycol;
b) from about 67.955% (w/w) to about 89.8999% (w/w) of petrolatum;
c) from about 0.1% (w/w) to about 1% (w/w) of butylated hydroxytoluene;
d) from about 0.0001% (w/w) to about 0.0450% (w/w) of edetate calcium disodium;
e) from about 3% (w/w) to about 10% (w/w) of a glyceride blend, wherein the glyceride blend comprises one or more monoglycerides and one or more diglycerides, wherein the one or more monoglycerides is in a total concentration of between 40% and 55% of the glyceride blend; and
f) from about 2% (w/w) to about 6% (w/w) of paraffin wax,
wherein the topical pharmaceutical formulation does not comprise an active agent and wherein the formulation comprises no more than about 0.5% (w/w) water.

In an exemplary embodiment, the topical pharmaceutical formulation comprises:
a) from about 8% (w/w) to about 10% (w/w) propylene glycol;
b) petrolatum;
c) an antioxidant;
d) a stabilizer;
e) an emulsifying agent; and
f) a stiffening agent,
wherein the topical pharmaceutical formulation does not comprise an active agent and wherein the formulation comprises no more than about 0.5% (w/w) water.

In an exemplary embodiment, the topical pharmaceutical formulation comprises:
a) from about 8% (w/w) to about 10% (w/w) propylene glycol;
b) petrolatum;
c) an antioxidant;
d) edetate calcium disodium;
e) an emulsifying agent; and
f) a stiffening agent,
wherein the topical pharmaceutical formulation does not comprise an active agent and wherein the formulation comprises no more than about 0.5% (w/w) water.

In an exemplary embodiment, the topical pharmaceutical formulation consists of:
a) from about 8% (w/w) to about 10% (w/w) propylene glycol;
b) petrolatum;
c) an antioxidant;
d) from about 0.0020% (w/w) to about 0.0040% (w/w) edetate calcium disodium;
e) an emulsifying agent; and
f) a stiffening agent.

In an exemplary embodiment, the topical pharmaceutical formulation consists essentially of:
a) from about 8% (w/w) to about 10% (w/w) propylene glycol;
b) petrolatum;
c) an antioxidant;
d) a stabilizer;
e) a glyceride blend, wherein the glyceride blend comprises one or more monoglycerides and one or more diglycerides; and
f) a stiffening agent
wherein the formulation comprises no more than about 0.5% (w/w) water.

In an exemplary embodiment, the topical pharmaceutical formulation consists essentially of:
a) from about 8% (w/w) to about 10% (w/w) propylene glycol;
b) petrolatum;
c) an antioxidant;
d) a stabilizer;
e) a glyceride blend, wherein the glyceride blend comprises one or more monoglycerides and one or more diglycerides, wherein the one or more monoglycerides is in a total concentration of between 40% and 55% of the glyceride blend; and
f) a stiffening agent
wherein the formulation comprises no more than about 0.5% (w/w) water.

In an exemplary embodiment, the topical pharmaceutical formulation consists essentially of:
a) from about 8% (w/w) to about 10% (w/w) propylene glycol;
b) petrolatum;
c) an antioxidant;
d) a stabilizer;
e) from about 5% (w/w) to about 10% (w/w) of a glyceride blend, wherein the glyceride blend comprises one or more monoglycerides and one or more diglycerides, wherein the one or more monoglycerides is in a total concentration of between 40% and 55% of the glyceride blend; and
f) a stiffening agent
wherein the formulation comprises no more than about 0.5% (w/w) water.

In an exemplary embodiment, the topical pharmaceutical formulation consists essentially of:
a) from about 8% (w/w) to about 10% (w/w) propylene glycol;
b) petrolatum;
c) an antioxidant;
d) a stabilizer;
e) from about 6% (w/w) to about 8% (w/w) of a glyceride blend, wherein the glyceride blend comprises one or more monoglycerides and one or more diglycerides, wherein the one or more monoglycerides is in a total concentration of between 40% and 55% of the glyceride blend; and
f) a stiffening agent
wherein the formulation comprises no more than about 0.5% (w/w) water.

In an exemplary embodiment, the topical pharmaceutical formulation consists essentially of:
a) from about 8% (w/w) to about 10% (w/w) propylene glycol;
b) petrolatum;
c) an antioxidant;
d) edetate calcium disodium;
e) from about 6% (w/w) to about 8% (w/w) of a glyceride blend, wherein the glyceride blend comprises one or more monoglycerides and one or more diglycerides, wherein the one or more monoglycerides is in a total concentration of between 40% and 55% of the glyceride blend; and
f) a stiffening agent
wherein the formulation comprises no more than about 0.5% (w/w) water.

In an exemplary embodiment, the topical pharmaceutical formulation consists essentially of:
a) from about 8% (w/w) to about 10% (w/w) propylene glycol;
b) petrolatum;
c) an antioxidant;
d) from about 0.0020% (w/w) to about 0.0040% (w/w) edetate calcium disodium;
e) from about 6% (w/w) to about 8% (w/w) of a glyceride blend, wherein the glyceride blend comprises one or more monoglycerides and one or more diglycerides, wherein the one or more monoglycerides is in a total concentration of between 40% and 55% of the glyceride blend; and
f) a stiffening agent
wherein the formulation comprises no more than about 0.5% (w/w) water.

In an exemplary embodiment, the topical pharmaceutical formulation consists essentially of:
a) from about 5% (w/w) to about 15% (w/w) propylene glycol;
b) petrolatum;
c) butylated hydroxytoluene;
d) edetate calcium disodium;
e) from about 5% (w/w) to about 10% (w/w) of a glyceride blend, wherein the glyceride blend comprises one or more monoglycerides and one or more diglycerides, wherein the one or more monoglycerides is in a total concentration of between 40% and 55% of the glyceride blend; and
f) a stiffening agent
wherein the formulation comprises no more than about 0.5% (w/w) water.

In an exemplary embodiment, the topical pharmaceutical formulation consists essentially of:
a) from about 5% (w/w) to about 15% (w/w) propylene glycol;
b) petrolatum;
c) butylated hydroxytoluene;
d) edetate calcium disodium;
e) from about 5% (w/w) to about 10% (w/w) of a glyceride blend, wherein the glyceride blend comprises one or more monoglycerides and one or more diglycerides, wherein the one or more monoglycerides is in a total concentration of between 40% and 55% of the glyceride blend; and
f) paraffin wax
wherein the formulation comprises no more than about 0.5% (w/w) water.

In an exemplary embodiment, the topical pharmaceutical formulation consists essentially of:
a) about 9% (w/w) propylene glycol;
b) white petrolatum;
c) about 0.1% (w/w) butylated hydroxytoluene;
d) about 0.0035% (w/w) edetate calcium disodium;
e) about 7% (w/w) of a glyceride blend, wherein the glyceride blend comprises one or more monoglycerides and one or more diglycerides, wherein the one or more monoglycerides is in a total concentration of between 40% and 55% of the glyceride blend; and
f) about 5% (w/w) paraffin wax.

In an exemplary embodiment, the topical pharmaceutical formulation consists of:
a) 9% (w/w) propylene glycol;
b) 78.8965% (w/w) white petrolatum;
c) 0.1% (w/w) butylated hydroxytoluene;
d) 0.0035% (w/w) edetate calcium disodium;
e) 7% (w/w) of a glyceride blend, wherein the glyceride blend comprises one or more monoglycerides and one or more diglycerides, wherein the one or more monoglycerides is in a total concentration of between 40% and 55% of the glyceride blend; and
f) 5% (w/w) paraffin wax
wherein the formulation comprises no more than about 0.5% (w/w) water.

Information regarding, excipients of use in the topical pharmaceutical formulations described herein, as well as making these topical pharmaceutical formulations, can be found herein as well as in Remington: The Science and Practice of Pharmacy, 21st Ed., Pharmaceutical Press (2011), the content of which is incorporated by reference for all purposes.

### III. The Methods

In another aspect of the invention, an active agent described herein, or a pharmaceutically acceptable salt, or a hydrate or a solvate thereof, can be utilized in the methods described herein. In another aspect of the invention, the topical pharmaceutical formulation described herein can be utilized in the methods described herein. In another aspect of the invention, the topical pharmaceutical formulation with an active agent, described herein, can be utilized in the methods described herein. In another aspect of the invention, the topical pharmaceutical formulation without an active agent, described herein, can be utilized in the methods described herein. In an exemplary embodiment, in any of the methods described herein, the animal being administered an active agent described herein, or a pharmaceutically acceptable salt, or a hydrate or a solvate thereof, or a topical pharmaceutical formulation described herein is not otherwise in need of treatment with said active agent described herein, or a pharmaceutically acceptable salt, or a hydrate or a solvate thereof, or the topical pharmaceutical formulation described herein. In an exemplary embodiment, in any of the methods described herein, the animal being administered an active agent described herein, or a pharmaceutically acceptable salt, or a hydrate or a solvate thereof, or a topical pharmaceutical formulation described herein is in need of treatment with said active agent described herein, or a pharmaceutically acceptable salt, or a hydrate or a solvate thereof, or a topical pharmaceutical formulation described herein. In an exemplary embodiment, in any of the methods described herein, the animal being administered a topical pharmaceutical formulation without an active agent, described herein, is not otherwise in need of treatment with the topical pharmaceutical formulation without an active agent. In an exemplary embodiment, in any of the methods described herein, the animal being administered a topical pharmaceutical formulation without an active agent, described herein, is in need of treatment with the topical pharmaceutical formulation without an active agent.

### III.a. Cytokine and/or Chemokine

In another aspect, the invention provides a method of decreasing the release of a cytokine and/or a chemokine, the method comprising contacting a cell with an active agent described herein, or a pharmaceutically acceptable salt, or a hydrate or a solvate thereof. In an exemplary embodiment, the invention provides a method of decreasing the release of a cytokine and/or a chemokine, the method comprising contacting a cell with a topical pharmaceutical formulation with an active agent described herein, or a pharmaceutically acceptable salt, or a hydrate or a solvate thereof. In an exemplary embodiment, the invention provides a method of decreasing the release of a cytokine and/or a chemokine, the method comprising contacting a cell with a topical pharmaceutical formulation without an active agent, described herein, or a pharmaceutically acceptable salt, or a hydrate or a solvate thereof. In an exemplary embodiment, for any of the methods provided herein, the release of the cytokine and/or chemokine is decreased. In an exemplary embodiment, for any of the methods described herein, the cytokine and/or chemokine is decreased.

In another aspect, the invention provides a method of decreasing the release of a cytokine and/or a chemokine from a cell, the method comprising contacting the cell with an active agent described herein, or a pharmaceutically acceptable salt, or a hydrate or a solvate thereof. In an exemplary embodiment, the active agent contacts the cell through administration of a topical pharmaceutical formulation described herein. In an exemplary embodiment, the invention provides a method of decreasing the release of a cytokine and/or a chemokine from a cell, the method comprising: contacting the cell with a topical pharmaceutical formulation without an active agent, described herein, or a pharmaceutically acceptable salt, or a hydrate or a solvate thereof. In an exemplary embodiment, the release of the cytokine and/or chemokine by the cell is decreased. In an exemplary embodiment, the cell is a skin cell.

In another aspect, the invention provides a method of decreasing the release of a cytokine and/or a chemokine from a cell, the method comprising contacting the cell with a topical pharmaceutical formulation without an active agent, described herein. In an exemplary embodiment, the release of the cytokine and/or chemokine by the cell is decreased. In an exemplary embodiment, the cell is a skin cell.

In another aspect, the invention provides a method of decreasing the release of a cytokine and/or a chemokine by a skin cell, the method comprising contacting the skin cell with an active agent by administering a topical pharmaceutical formulation described herein. In an exemplary embodiment, the release of the cytokine and/or chemokine by the skin cell is decreased.

In another aspect, the invention provides a method of decreasing the release of a cytokine and/or a chemokine by a skin cell, the method comprising contacting the skin cell with a topical pharmaceutical formulation described herein. In an exemplary embodiment, the release of the cytokine and/or chemokine by the skin cell is decreased.

In another aspect, the invention provides a method of decreasing the release of a cytokine and/or a chemokine by a skin cell, the method comprising contacting the skin cell with a topical pharmaceutical formulation without an active agent, described herein. In an exemplary embodiment, the release of the cytokine and/or chemokine by the skin cell is decreased.

In an exemplary embodiment, the cytokine and/or chemokine is selected from the group consisting of TNF-α, IFN-γ, IL-2, IL-4, IL-5, IL-13, IL-22, IL-23, and IL-31. In an exemplary embodiment, the cytokine and/or chemokine is TNF-α. In an exemplary embodiment, the cytokine and/or chemokine is IL-23. In an exemplary embodiment, the cytokine and/or chemokine is IL-2. In an exemplary embodiment, the cytokine and/or chemokine is IL-17.

In an exemplary embodiment, for any of the methods described herein, the active agent or the topical pharmaceutical formulation is present in an amount which decreases the release of a cytokine and/or chemokine described herein by at least about 5 to about 100%, or at least about 30 to about 100%, 40 to about 100%, or at least about 50 to about 100%, or at least about 60 to about 100%, or at least about 70 to about 100%, or at least about 80 to about 100%, or at least about 90 to about 100%, or at least about 30 to about 70%, or at least about 40 to about 90%, or at least about 45 to about 80%, or at least about 55 to about 75%, or at least about 75 to about 98%, or at least about 55 to about 99%, or at least about 5% to about 20% or at least about 10% to about 25%.

### III.b. Phosphodiesterase

In another aspect, the invention provides a method of inhibiting a phosphodiesterase (PDE), the method comprising contacting the phosphodiesterase with an active agent described herein, or a pharmaceutically acceptable salt, or a hydrate or a solvate thereof. In an exemplary embodiment, the compound of the invention is a compound described herein or a pharmaceutically acceptable salt thereof. In an exemplary embodiment, the compound of the invention is a compound described herein. In an exemplary embodiment, the amount of the compound is a therapeutically effective amount. In an exemplary embodiment, the compound is according to a formula described herein. In an exemplary embodiment, for any of the methods described herein, the phosphodiesterase is inhibited.

In another aspect, the invention provides a method of inhibiting a phosphodiesterase (PDE) in a cell, the method comprising contacting the cell with a topical pharmaceutical formulation with an active agent described herein, or a pharmaceutically acceptable salt, or a hydrate or a solvate thereof. In another aspect, the invention provides a method of inhibiting a phosphodiesterase (PDE) in a cell, the method comprising contacting the cell with a topical pharmaceutical formulation without an active agent described herein, or a pharmaceutically acceptable salt, or a hydrate or a solvate thereof. In an exemplary embodiment, the amount of the active agent is a therapeutically effective amount. In an exemplary embodiment, the amount of the topical pharmaceutical formulation with an active agent is a therapeutically effective amount. In an exemplary embodiment, the amount of the topical pharmaceutical formulation without an active agent is a therapeutically effective amount. In an exemplary embodiment, for any of the methods described herein, the cell is a skin cell. In an exemplary embodiment, for any of the methods described herein, the phosphodiesterase is inhibited.

In an exemplary embodiment, the phosphodiesterase is selected from the group consisting of PDE1, PDE2, PDE3, PDE4, PDE5, PDE6, PDE7, PDE8, PDE9, PDE10 and PDE11. In an exemplary embodiment, the phosphodiesterase is PDE4. In an exemplary embodiment, the PDE4 is selected from the group consisting of PDE4A, PDE4B, PDE4C and PDE4D. In an exemplary embodiment, the PDE4 is PDE4B. In an exemplary embodiment, the phosphodiesterase is PDE7.

In an exemplary embodiment, the invention provides a method for inhibiting a phosphodiesterase4 (PDE4), but not significantly inhibiting at least one PDE which is selected from the group consisting of PDE1, PDE2, PDE3, PDE5 and PDE6, involving contacting a cell with a topical pharmaceutical formulation described herein, thereby providing said inhibition.

In an exemplary embodiment, for any of the methods described herein, the active agent or the topical pharmaceutical formulation is present in an amount which inhibits a phosphodiesterase described herein by at least about 5 to about 100%, or at least about 30 to about 100%, 40 to about 100%, or at least about 50 to about 100%, or at least about 60 to about 100%, or at least about 70 to about 100%, or at least about 80 to about 100%, or at least about 90 to about 100%, or at least about 30 to about 70%, or at least about 40 to about 90%, or at least about 45 to about 80%, or at least about 55 to about 75%, or at least about 75 to about 98%, or at least about 55 to about 99%, or at least about 5% to about 20% or at least about 10% to about 25%.

### III.c. Conditions

In another aspect, the invention provides a method of treating and/or preventing a condition in an animal, the method comprising administering to the animal a therapeutically effective and/or prophylactically effective amount of a topical pharmaceutical formulation with an active agent, described herein. In an exemplary embodiment, the condition is treated and/or prevented. In an exemplary embodiment, the animal is in need of treatment and/or prophylaxis thereof. In an exemplary embodiment, the animal is not otherwise in need of treatment and/or prophylaxis thereof. In an exemplary embodiment, the condition is a condition of the skin. In an exemplary embodiment, the condition is pruritis.

In another aspect, the invention provides a method of treating and/or preventing a condition in an animal, the method comprising administering to the animal, a therapeutically effective and/or prophylactically effective amount of a topical pharmaceutical formulation without an active agent, described herein. In an exemplary embodiment, the condition is treated and/or prevented. In an exemplary embodiment, the animal is in need of treatment and/or prophylaxis thereof. In an exemplary embodiment, the condition is a condition of the skin. In an exemplary embodiment, the condition is pruritis.

In another aspect, the invention provides a method of treating and/or preventing an inflammatory-related condition in an animal, the method comprising administering to the animal a therapeutically effective and/or prophylactically effective amount of a topical pharmaceutical formulation with an active agent, described herein. In an exemplary embodiment, the inflammatory-related condition is treated and/or prevented. In an exemplary embodiment, the animal is in need of treatment and/or prophylaxis thereof. In an exemplary embodiment, the animal is not otherwise in need of treatment and/or prophylaxis thereof. In an exemplary embodiment, the inflammatory-related condition is a condition of the skin.

In another aspect, the invention provides a method of treating and/or preventing an inflammatory-related condition in an animal, the method comprising administering to the animal, a therapeutically effective and/or prophylactically effective amount of a topical pharmaceutical formulation without an active agent, described herein. In an exemplary embodiment, the inflammatory-related condition is treated and/or prevented. In an exemplary embodiment, the animal is in need of treatment and/or prophylaxis thereof.

In an exemplary embodiment, the inflammatory-related condition is psoriasis. In an exemplary embodiment, the inflammatory-related condition is plaque psoriasis or flexural psoriasis (inverse psoriasis) or guttate psoriasis or pustular psoriasis or nail psoriasis or psoriatic arthritis or erythrodermic psoriasis. In an exemplary embodiment, the inflammatory-related condition is plaque psoriasis. In an exemplary embodiment, the inflammatory-related condition is nail psoriasis.

In an exemplary embodiment, the inflammatory-related condition is dermatitis. In an exemplary embodiment, the inflammatory-related condition is contact dermatitis or atopic dermatitis or nummular dermatitis or seborrheic dermatitis or stasis dermatitis. In an exemplary embodiment, the inflammatory-related condition is atopic dermatitis. In an exemplary embodiment, the inflammatory-related condition is eczema.

In an exemplary embodiment, for any of the methods described herein, the animal is selected from the group consisting of human, cattle, deer, reindeer, goat, honey bee, pig, sheep, horse, cow, bull, dog, guinea pig, gerbil, rabbit, cat, camel, yak, elephant, ostrich, otter, chicken, duck, goose, guinea fowl, pigeon, swan, and turkey. In another exemplary embodiment, for any of the methods described herein, the animal is selected from the group consisting of a human, cattle, goat, pig, sheep, horse, cow, bull, dog, guinea pig, gerbil, rabbit, cat, chicken and turkey. In another exemplary embodiment, for any of the methods described herein, the animal is a human.

In another exemplary embodiment, the method involves preventing psoriasis by administering a topical pharmaceutical formulation with an active agent, described herein, to an animal. In an exemplary embodiment, the psoriasis is prevented. In another exemplary embodiment, the method involves preventing psoriasis by administering a topical pharmaceutical formulation without an active agent, described herein, to an animal. In an exemplary embodiment, the psoriasis is prevented. In another exemplary embodiment, the method involves treating psoriasis by administering a topical pharmaceutical formulation with an active agent, described herein, to an animal. In an exemplary embodiment, the psoriasis is treated.

In another exemplary embodiment, the method involves treating psoriasis by administering a topical pharmaceutical formulation without an active agent, described herein, to an animal. In an exemplary embodiment, the psoriasis is treated.

In another exemplary embodiment, the method involves preventing plaque psoriasis by administering a topical pharmaceutical formulation with an active agent, described herein, to an animal. In an exemplary embodiment, the plaque psoriasis is prevented.

In another exemplary embodiment, the method involves preventing plaque psoriasis by administering a topical pharmaceutical formulation without an active agent, described herein, to an animal. In an exemplary embodiment, the plaque psoriasis is prevented.

In another exemplary embodiment, the method involves treating plaque psoriasis by administering a topical pharmaceutical formulation with an active agent, described herein, to an animal. In an exemplary embodiment, the plaque psoriasis is treated. In another exemplary embodiment, the method involves treating plaque psoriasis by administering a topical pharmaceutical formulation without an active agent, described herein, to an animal. In an exemplary embodiment, the plaque psoriasis is treated.

In another exemplary embodiment, the method involves preventing nail psoriasis by administering a topical pharmaceutical formulation with an active agent, described herein, to an animal. In an exemplary embodiment, the nail psoriasis is prevented.

In another exemplary embodiment, the method involves preventing nail psoriasis by administering a topical pharmaceutical formulation without an active agent, described herein, to an animal. In an exemplary embodiment, the nail psoriasis is prevented. In another exemplary embodiment, the method involves treating nail psoriasis by administering a topical pharmaceutical formulation with an active agent, described herein, to an animal. In an exemplary embodiment, the nail psoriasis is treated. In another exemplary embodiment, the method involves treating nail psoriasis by administering a topical pharmaceutical formulation without an active agent, described herein, to an animal. In an exemplary embodiment, the nail psoriasis is treated.

In another exemplary embodiment, the method involves preventing atopic dermatitis by administering a topical pharmaceutical formulation with an active agent, described herein, to an animal. In an exemplary embodiment, the atopic dermatitis is prevented.

In another exemplary embodiment, the method involves preventing atopic dermatitis by administering a topical pharmaceutical formulation without an active agent, described herein, to an animal. In an exemplary embodiment, the atopic dermatitis is prevented.

In another exemplary embodiment, the method involves treating atopic dermatitis by administering a topical pharmaceutical formulation with an active agent, described herein, to an animal. In an exemplary embodiment, the atopic dermatitis is treated.

In another exemplary embodiment, the method involves treating atopic dermatitis by administering a topical pharmaceutical formulation without an active agent, described herein, to an animal. In an exemplary embodiment, the atopic dermatitis is treated.

Exemplary embodiments are summarized herein below.

In an exemplary embodiment, the invention provides a topical pharmaceutical formulation comprising: a) an active agent which treats an inflammatory-related condition, or a pharmaceutically acceptable salt, or a hydrate or a solvate thereof; b) from about 5% (w/w) to about 15% (w/w) propylene glycol; and c) petrolatum.

In an exemplary embodiment, according to the above paragraph, the formulation comprises no more than about 0.5% (w/w) water.

In an exemplary embodiment, according to any of the above paragraphs, the amount of the propylene glycol is from about 7% (w/w) to about 11% (w/w).

In an exemplary embodiment, according to any of the above paragraphs, the amount of the propylene glycol is from about 6% (w/w) to about 10% (w/w). In an exemplary embodiment, according to any of the above paragraphs, the amount of the propylene glycol is from about 8% (w/w) to about 10% (w/w).

In an exemplary embodiment, according to any of the above paragraphs, the amount of the propylene glycol is about 9% (w/w).

In an exemplary embodiment, according to any of the above paragraphs, the amount of the propylene glycol is 9% (w/w).

In an exemplary embodiment, according to any of the above paragraphs, the propylene glycol is propylene glycol USP.

In an exemplary embodiment, according to any of the above paragraphs, the topical pharmaceutical formulation further comprises an emulsifying agent.

In an exemplary embodiment, according to any of the above paragraphs, wherein the emulsifying agent is a glyceride blend.

In an exemplary embodiment, according to any of the above paragraphs, wherein the emulsifying agent is a glyceride blend, and the glyceride blend comprises a monoglyceride and a diglyceride.

In an exemplary embodiment, according to any of the above paragraphs, the amount of the glyceride blend is from about 3% (w/w) to about 10% (w/w).

In an exemplary embodiment, according to any of the above paragraphs, the amount of the glyceride blend is from about 5% (w/w) to about 8% (w/w).

In an exemplary embodiment, according to any of the above paragraphs, the amount of the glyceride blend is from about 6% (w/w) to about 8% (w/w).

In an exemplary embodiment, according to any of the above paragraphs, the amount of the glyceride blend is about 7% (w/w).

In an exemplary embodiment, according to any of the above paragraphs, the amount of the glyceride blend is 7% (w/w). In an exemplary embodiment, according to any of the above paragraphs, the glyceride blend is is Mono- and Di-glyceride NF.

In an exemplary embodiment, according to any of the above paragraphs, from about 40% (w/w) to about 55% (w/w) of the glyceride blend is a monoglyceride.

In an exemplary embodiment, according to any of the above paragraphs, the topical pharmaceutical formulation further comprises a stabilizer. In an exemplary embodiment, according to any of the above paragraphs, the stabilizer is ethylenediaminetetraacetic acid, or a pharmaceutically acceptable salt thereof. In an exemplary embodiment, according to any of the above paragraphs, further comprising a sodium salt or a potassium salt or a calcium salt, or a mixture thereof, of ethylenediaminetetraacetic acid. In an exemplary embodiment, according to any of the above paragraphs, further comprising a sodium salt or a calcium salt, or a mixture thereof, of ethylenediaminetetraacetic acid.

In an exemplary embodiment, according to any of the above paragraphs, further comprising edetate calcium disodium.

In an exemplary embodiment, according to any of the above paragraphs, further comprising edetate calcium disodium USP.

In an exemplary embodiment, according to any of the above paragraphs, the amount of the ethylenediaminetetraacetic acid, or a pharmaceutically acceptable salt thereof, is from about 0.0001% (w/w) to about 0.01% (w/w). In an exemplary embodiment, according to any of the above paragraphs, the amount of the ethylenediaminetetraacetic acid, or a pharmaceutically acceptable salt thereof, is from about 0.001% (w/w) to about 0.01% (w/w).

In an exemplary embodiment, according to any of the above paragraphs, the amount of the ethylenediaminetetraacetic acid, or a pharmaceutically acceptable salt thereof, is from about 0.001% (w/w) to about 0.005% (w/w).

In an exemplary embodiment, according to any of the above paragraphs, the amount of the ethylenediaminetetraacetic acid, or a pharmaceutically acceptable salt thereof, is from about 0.0025% (w/w) to about 0.0045% (w/w).

In an exemplary embodiment, according to any of the above paragraphs, the amount of the ethylenediaminetetraacetic acid, or a pharmaceutically acceptable salt thereof, is about 0.0035% (w/w).

In an exemplary embodiment, according to any of the above paragraphs, the amount of the ethylenediaminetetraacetic acid, or a pharmaceutically acceptable salt thereof, is 0.0035% (w/w).

In an exemplary embodiment, according to any of the above paragraphs, the amount of the edetate calcium disodium USP is 0.0035% (w/w).

In an exemplary embodiment, according to any of the above paragraphs, further comprising an antioxidant.

In an exemplary embodiment, according to any of the above paragraphs, the antioxidant is selected from the group consisting of butylated hydroxytoluene, ascorbic acid or a pharmaceutically acceptable salt thereof, ascorbic palmitate, butylated hydroxyanisole, 2,4,5-trihydroxybutyrophenone, 4-hydroxymethyl-2,6-di-tertbutylphenol, erythorbic acid, gum guaiac, propyl gallate, thiodipropionic acid, dilauryl thiodipropionate, tert-butylhydroquinone and tocopherols such as vitamin E, and the like, including pharmaceutically acceptable salts and esters thereof, and mixtures thereof.

In an exemplary embodiment, according to any of the above paragraphs, the antioxidant is butylated hydroxytoluene.

In an exemplary embodiment, according to any of the above paragraphs, the amount of the antioxidant is from about 0.01% (w/w) to about 1% (w/w).

In an exemplary embodiment, according to any of the above paragraphs, the amount of the antioxidant is from about 0.05% (w/w) to about 0.5% (w/w).

In an exemplary embodiment, according to any of the above paragraphs, the amount of the antioxidant is about 0.1% (w/w).

In an exemplary embodiment, according to any of the above paragraphs, the amount of the antioxidant is 0.1% (w/w).

In an exemplary embodiment, according to any of the above paragraphs, the antioxidant is butylated hydroxytoluene.

In an exemplary embodiment, according to any of the above paragraphs, the antioxidant is butylated hydroxytoluene NF.

In an exemplary embodiment, according to any of the above paragraphs, further comprising a stiffening agent.

In an exemplary embodiment, according to any of the above paragraphs, the amount of the stiffening agent is from about 2% (w/w) to about 8% (w/w).

In an exemplary embodiment, according to any of the above paragraphs, the amount of the stiffening agent is from about 4% (w/w) to about 6% (w/w). In an exemplary embodiment, according to any of the above paragraphs, the amount of the stiffening agent is about 5% (w/w).

In an exemplary embodiment, according to any of the above paragraphs, the amount of the stiffening agent is 5% (w/w).

In an exemplary embodiment, according to any of the above paragraphs, the stiffening agent is selected from the group consisting of beeswax, paraffin wax, wax, and spermaceti wax.

In an exemplary embodiment, according to any of the above paragraphs, the stiffening agent is paraffin wax.

In an exemplary embodiment, according to any of the above paragraphs, the stiffening agent is paraffin wax NF.

In an exemplary embodiment, according to any of the above paragraphs, the stiffening agent is 5% (w/w) paraffin wax NF.

In an exemplary embodiment, according to any of the above paragraphs, the active agent is 5-(4-cyanophenoxy)-1,3-dihydro-1-hydroxy-2,1-benzoxaborole. In an exemplary embodiment, according to any of the above paragraphs, the amount of the active agent is from about 0.1% (w/w) to about 2.0% (w/w).

In an exemplary embodiment, according to any of the above paragraphs, the amount of the 5-(4-cyanophenoxy)-1,3-dihydro-1-hydroxy-2,1-benzoxaborole is from about 0.1% (w/w) to about 2.0% (w/w).

In an exemplary embodiment, according to any of the above paragraphs, the amount of the 5-(4-cyanophenoxy)-1,3-dihydro-1-hydroxy-2,1-benzoxaborole is 2.0% (w/w).

In an exemplary embodiment, according to any of the above paragraphs, the remainder of the formulation is petrolatum. In an exemplary embodiment, according to any of the above paragraphs, the remainder of the formulation is White Petrolatum.

In an exemplary embodiment, according to any of the above paragraphs, the remainder of the formulation is White Petrolatum USP.

In an exemplary embodiment, the topical pharmaceutical formulation consists of: a) 5-(4-cyanophenoxy)-1,3-dihydro-1-hydroxy-2,1-benzoxaborole; b) propylene glycol; c) butylated hydroxytoluene; d) edetate calcium disodium; e) mono- and di- glycerides; f) paraffin wax; and g) white petrolatum.

In an exemplary embodiment, the topical pharmaceutical formulation consists essentially of: a) 5-(4-cyanophenoxy)-1,3-dihydro-1-hydroxy-2,1-benzoxaborole; b) propylene glycol; c) butylated hydroxytoluene; d) edetate calcium disodium; e) mono- and di- glycerides; f) paraffin wax; and g) white petrolatum.

In an exemplary embodiment, the topical pharmaceutical formulation comprises: a) 5-(4-cyanophenoxy)-1,3-dihydro-1-hydroxy-2,1-benzoxaborole; b) propylene glycol; c) butylated hydroxytoluene; d) edetate calcium disodium; e) mono- and di- glycerides; f) paraffin wax; and g) white petrolatum.

In an exemplary embodiment, the topical pharmaceutical formulation consists of: a) 2% (w/w) 5-(4-cyanophenoxy)-1,3-dihydro-1-hydroxy-2,1-benzoxaborole; b) 9% (w/w) propylene glycol; c) 0.1% (w/w) butylated hydroxytoluene; d) 0.0035% (w/w) edetate calcium disodium; e) 7% (w/w) mono- and di- glycerides; f) 5% (w/w) paraffin wax; and g) 76.8965% (w/w) white petrolatum.

In an exemplary embodiment, the topical pharmaceutical formulation consists essentially of: a) 2% (w/w) 5-(4-cyanophenoxy)-1,3-dihydro-1-hydroxy-2,1-benzoxaborole; b) 9% (w/w) propylene glycol; c) 0.1% (w/w) butylated hydroxytoluene; d) 0.0035% (w/w) edetate calcium disodium; e) 7% (w/w) mono- and di- glycerides, wherein between 40% and 55% is the monoglyceride; f) 5% (w/w) paraffin wax; and g) 76.8965% (w/w) white petrolatum.

In an exemplary embodiment, the topical pharmaceutical formulation consists essentially of: a) about 2% (w/w) 5-(4-cyanophenoxy)-1,3-dihydro-1-hydroxy-2,1-benzoxaborole; b) about 9% (w/w) propylene glycol; c) about 0.1% (w/w) butylated hydroxytoluene; d) about 0.0035% (w/w) edetate calcium disodium; e) about 7% (w/w) mono- and di- glycerides, wherein between 40% and 55% is the monoglyceride; f) about 5% (w/w) paraffin wax; and g) about 76.8965% (w/w) white petrolatum.

In an exemplary embodiment, the topical pharmaceutical formulation consists of: a) about 2% (w/w) 5-(4-cyanophenoxy)-1,3-dihydro-1-hydroxy-2,1-benzoxaborole; b) about 9% (w/w) propylene glycol; c) about 0.1% (w/w) butylated hydroxytoluene; d) about 0.0035% (w/w) edetate calcium disodium; e) about 7% (w/w) mono- and di-glycerides, wherein between 40% and 55% is the monoglyceride; f) about 5% (w/w) paraffin wax; and g) about 76.8965% (w/w) white petrolatum.

In an exemplary embodiment, the invention is a method of decreasing the release of a cytokine and/or a chemokine, the method comprising contacting a cell with the topical pharmaceutical formulation according to any of the above paragraphs.

In an exemplary embodiment, the invention is a method of treating an inflammatory-related condition in an animal, the method comprising administering to the animal a therapeutically effective amount of the topical pharmaceutical formulation according to any of the above paragraphs.

In an exemplary embodiment, according to any of the above method paragraphs, the inflammatory-related condition is psoriasis.

In an exemplary embodiment, according to any of the above method paragraphs, the inflammatory-related condition is atopic dermatitis.

In an exemplary embodiment, according to any of the above method paragraphs, the animal is a human.

In an exemplary embodiment, the invention is a method of treating atopic dermatitis in a human, the method comprising administering to the human a therapeutically effective amount of the topical pharmaceutical formulation according to any of the above paragraphs.

In an exemplary embodiment, the invention is a method of treating psoriasis in a human, the method comprising administering to the human a therapeutically effective amount of the topical pharmaceutical formulation according to any of the above paragraphs.

The invention is further illustrated by the Examples that follow. The Examples are not intended to define or limit the scope of the invention.

### EXPERIMENTALS

### Polymorphic Studies

Different crystalline solid forms of the same compound often possess different solid-state properties such as melting point, solubility, dissolution rate, hygroscopicity, powder flow, mechanical properties, chemical stability and physical stability. These solid-state properties may offer advantages in filtration, drying, and dosage form manufacturing unit operations. Thus, once different crystalline solid forms of the same compound have been identified, the optimum crystalline solid form under any given set of processing and manufacturing conditions may be determined as well as the different solid-state properties of each crystalline solid form.

Polymorphs of a molecule can be obtained by a number of methods known in the art. Such methods include, but are not limited to, melt recrystallization, melt cooling, solvent recrystallization, desolvation, rapid evaporation, rapid cooling, slow cooling, vapor diffusion and sublimation. Polymorphs can be detected, identified, classified and characterized using well-known techniques such as, but not limited to, differential scanning calorimetry (DSC), thermogravimetry (TGA), X-ray powder diffractometry (XRPD), single crystal X-ray diffractometry, solid state nuclear magnetic resonance (NMR), infrared (IR) spectroscopy, Raman spectroscopy, and hot-stage optical microscopy.

During the development of the manufacturing process for crisaborole drug substance, attemps were directed towards identifying the polymorphs of crisaborole. Several solvent systems that involved polar protic solvents like water, polar aprotic sovletns like dimethoxyethane and nonpolar solvents such as heptanes were explored. These studies resulted in the identification of three polymorphs of crisaborole drug substance that were differentiated by X-ray Powder Diffraction. Form 2 was the form utilized in clinical studies through Phase 2. Form 1 was identified as the commercial form and was utilized in Phase 3 studies.

There are a number of analytical methods one of ordinary skill in the art in solid-state chemistry can use to analyze solid forms. The term "analyze" as used herein means to obtain information about the solid-state structure of solid forms. For example, X-ray powder diffraction is a suitable technique for differentiating amorphous solid forms from crystalline solid forms and for characterizing and identifying crystalline solid forms of a compound. X-ray powder diffraction is also suitable for quantifying the amount of a crystalline solid form (or forms) in a mixture. In X-ray powder diffraction, X-rays are directed onto a crystal and the intensity of the diffracted X-rays is measured as a function of twice the angle between the X-ray source and the beam diffracted by the sample. The intensity of these diffracted X-rays can be plotted on a graph as peaks with the x-axis being twice the angle (this is known as the "2θ" angle) between the X-ray source and the diffracted X-rays and with the y-axis being the intensity of the diffracted X-rays. This graph is called an X-ray powder diffraction pattern or powder pattern. Different crystalline solid forms exhibit different powder patterns because the location of the peaks on the x-axis is a property of the solid-state structure of the crystal.

Such powder patterns, or portions thereof, can be used as an identifying fingerprint for a crystalline solid form. Thus, one could take a powder pattern of an unknown sample and compare that powder pattern with a reference powder pattern. A positive match would mean that the unknown sample is of the same crystalline solid form as that of the reference. One could also analyze an unknown sample containing a mixture of solid forms by adding and subtracting powder patterns of known compounds.

When selecting peaks in a powder pattern to characterize a crystalline solid form or when using a reference powder pattern to identify a form, one identifies a peak or collection of peaks in one form that are not present in the other solid forms.

The term "characterize" as used herein means to select an appropriate set of data capable of distinguishing one solid form from another. That set of data in X-ray powder diffraction is the position of one or more peaks. Selecting which X-ray powder diffraction peaks define a particular form is said to characterize that form.

The term "identify" as used herein means taking a selection of characteristic data for a solid form and using those data to determine whether that form is present in a sample. In X-ray powder diffraction, those data are the x-axis positions of the one or more peaks characterizing the form in question as discussed above. For example, once one determines that a select number of X-ray diffraction peaks characterize a particular solid form, one can use those peaks to determine whether that form is present in a sample.

When characterizing and/or identifying crystalline solid forms of the same chemical compound with X-ray powder diffraction, it is often not necessary to use the entire powder pattern. A smaller subset of the entire powder pattern can often be used to perform the characterization and/or identification. By selecting a collection of peaks that differentiate the crystalline solid form from other crystalline solid forms of the compound, one can rely on those peaks to both characterize the form and to identify the form in, for example, an unknown mixture. Additional data can be added, such as from another analytical technique or additional peaks from the powder pattern, to characterize and/or identify the form should, for instance, additional polymorphs be identified later.

Due to differences in instruments, samples, and sample preparation, peak values is sometimes reported with the modifier "about" in front of the peak values. This is common practice in the solid-state chemical arts because of the variation inherent in peak values. A typical precision of the 2θ x-axis value of a peak in a powder pattern is on the order of plus or minus 0.2° 2θ. Thus, a powder diffraction peak that appears at "about 9.2° 2θ," means that the peak could be between 9.0° 2θ and 9.4° 2θ when measured on most X-ray diffractometers under most conditions. Variability in peak intensity is a result of how individual crystals are oriented in the sample container with respect to the external X-ray source (known as "preferred orientation"). This orientation effect does not provide structural information about the crystal. X-ray powder diffraction is just one of several analytical techniques one may use to characterize and/or identify crystalline solid forms. Spectroscopic techniques such as Raman (including microscopic Raman), infrared, and solid state NMR spectroscopies may be used to characterize and/or identify crystalline solid forms. These techniques may also be used to quantify the amount of one or more crystalline solid forms in a mixture and peak values can also be reported with the modifier "about" in front of the peak values. A typical variability for a peak value associated with an FT-Raman and FT-Infrared measurement is on the order of plus or minus 2 cm⁻¹. A typical variability for a peak value associated with a ¹³C chemical shift is on the order of plus or minus 0.2 ppm for crystalline material. A typical variability for a value associated with a differential scanning calorimetry onset temperature is on the order of plus or minus 5° C.

The term "room temperature" as used herein refers to the temperature range of 20 °Cto 23 °C..

### 1. Instrument Method (API and Drug Product Scans)

Powder X-ray diffraction analysis was conducted using a Bruker AXS D8 ADVANCE diffractometer equipped with a Cu radiation source (K-α average). The system is equipped with a 2.5 axial Soller slits on the primary side. The secondary side utilizes 2.5 axial Soller slits and motorized slits. Diffracted radiation was detected by a Lynx Eye XE detector. The X-ray tube voltage and amperage were set to 40 kV and 40 mA respectively. Data was collected in the Theta-Theta goniometer at the Cu wavelength from 3.0 to 40.0 degrees 2-Theta using a step size of 0.037 degrees and a step time of 1920 seconds. Samples were prepared by placing them in a low background holder and rotated during collection. Data were collected using Bruker DIFFRAC Plus software (Version 9.0.0.2) and analysis was performed by EVA diffract plus software.

The PXRD data file was not processed prior to peak searching. Using the peak search algorithm in the EVA software, peaks selected with a threshold value of 1 and a width value of 0.3 were used to make preliminary peak assignments. The output of automated assignments was visually checked to ensure validity and adjustments were manually made if necessary. Peaks with relative intensity of ≥ 2% were generally chosen. The peaks which were not resolved or were consistent with noise were not selected. A typical error associated with the peak position from PXRD stated in USP up to +/- 0.2° 2-Theta (USP-941). The peak fitting associated with Forms 1, 2 and 3 are listed in Tables 1-3; Table 4 lists the peaks for Form 1 that can be distinguished from the placebo drug product. For example, 6.0, 12.1, 14.1 and 15.4 peaks are associated with crisaborole. In particular, peaks 6.0 and 15.4 are preferred associated peaks.

**Table 1: PXRD peak list for Form 1 API. Asterisked peak positions represent characteristic peaks.**

| **Angle** | **Rel. Intensity** |
|---|---|
| 6.1* | 100 |
| 12.2 | 14 |
| 14.2 | 9 |
| 15.4* | 31 |
| 16.1 | 6 |
| 17.7 | 2 |
| 18.2 | 46 |
| 21.5 | 11 |
| 23.1 | 5 |
| 24.3 | 8 |
| 24.9 | 12 |
| 26.2 | 61 |
| 26.5 | 7 |
| 28.5 | 12 |
| 29.1 | 3 |
| 31.6 | 7 |
| 31.4 | 17 |
| 31.7 | 7 |
| 32.8 | 2 |
| 33.8 | 3 |
| 37.0 | 2 |

**Table 2: PXRD peak list for Form 2 API**

| **Angle 2Theta°** | **Relative Intensity %** |
|---|---|
| 7.1 | 6 |
| 12.3 | 12 |
| 14.3 | 18 |
| 14.9 | 4 |
| 15.5 | 2 |
| 16.4 | 13 |
| 16.7 | 75 |
| 17.7 | 43 |
| 17.9 | 7 |
| 18.4 | 10 |
| 20.0 | 8 |
| 20.9 | 100 |
| 21.4 | 29 |
| 21.8 | 36 |
| 22.2 | 5 |
| 22.7 | 59 |
| 23.2 | 30 |
| 23.5 | 8 |
| 24.2 | 4 |
| 24.9 | 31 |
| 24.9 | 26 |
| 26.1 | 11 |
| 26.4 | 7 |
| 26.5 | 7 |
| 27.1 | 8 |
| 27.5 | 9 |
| 27.8 | 15 |
| 28.0 | 32 |
| 28.8 | 6 |
| 29.1 | 3 |
| 30.1 | 10 |
| 31.0 | 3 |
| 31.5 | 5 |
| 32.1 | 3 |
| 33.7 | 3 |
| 34.4 | 2 |
| 34.9 | 5 |
| 37.4 | 7 |
| 38.9 | 2 |
| 39.9 | 2 |
| 43.1 | 4 |
| 45.6 | 2 |
| 46.5 | 3 |

**Table 3: PXRD peak list for crisaborole Form 3 API.**

| **Angle 2-Theta°** | **Rel. Intensity %** |
|---|---|
| 13.8 | 2 |
| 14.3 | 2 |
| 15.8 | 5 |
| 16.4 | 6 |
| 16.6 | 13 |
| 16.7 | 13 |
| 16.8 | 7 |
| 17.7 | 8 |
| 18.4 | 9 |
| 18.4 | 8 |
| 18.8 | 15 |
| 19.7 | 12 |
| 20.8 | 100 |
| 21.4 | 13 |
| 21.9 | 9 |
| 22.7 | 16 |
| 23.2 | 4 |
| 23.5 | 4 |
| 23.9 | 17 |
| 24.9 | 4 |
| 26.4 | 3 |
| 26.2 | 8 |
| 26.5 | 3 |
| 27.1 | 3 |
| 27.6 | 6 |
| 27.9 | 33 |
| 28.8 | 5 |
| 29.1 | 3 |
| 31.4 | 6 |
| 32.3 | 2 |
| 34.9 | 2 |
| 37.3 | 6 |

**Table 4: PXRD peak list for Crisaborole Form 1 drug product.**

| **Angle 2-Theta°** | **Relative Intensity %** |
|---|---|
| 6.0 | 89 |
| 12.1 | 11 |
| 14.1 | 33 |
| 15.4 | 100 |
| 16.0 | 17 |
| 17.7 | 6 |
| 18.2 | 40 |
| 18.6 | 2 |
| 21.5 | 33 |
| 23.1 | 6 |
| 24.9 | 37 |
| 26.2 | 87 |
| 26.5 | 14 |
| 27.6 | 2 |
| 28.5 | 33 |
| 28.5 | 27 |
| 29.1 | 7 |
| 31.0 | 6 |
| 31.4 | 11 |
| 31.7 | 9 |
| 31.8 | 8 |
| 32.8 | 3 |
| 33.8 | 7 |
| 37.1 | 3 |
| 39.3 | 2 |

### 2. Method for Producing Form 1

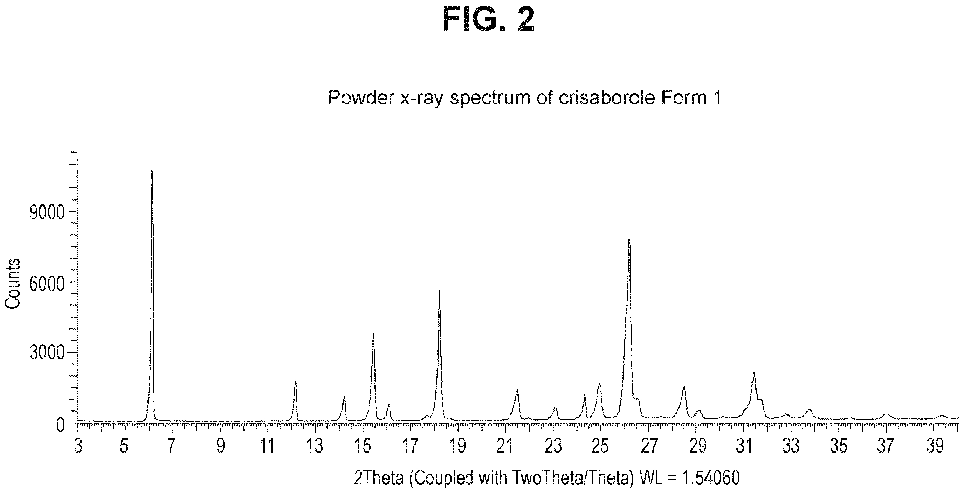

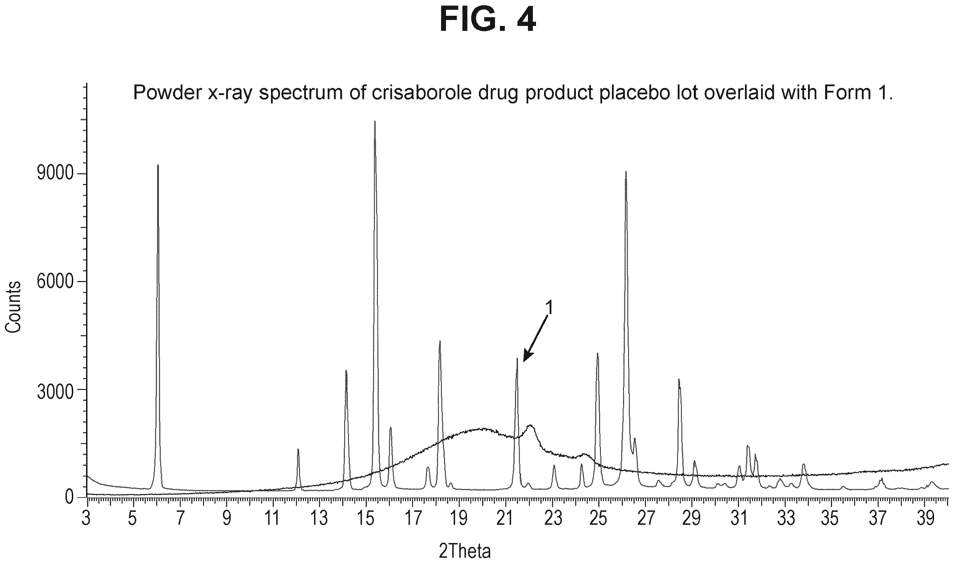

Crisaborole was dissolved in acetic acid (∼3.5 vol) at 70°C followed by the addition of about 0.75 vol water. The mixture was cooled to ∼ 61°C (range: between 58°C and 63°C), seeded with crisaborole (1% seeding ± 1%) and maintained at that temperature for approximately 15 minutes. The reaction mixture was then cooled to 50°C (± 3°C) over a 45 to 60 minute period and monitored by Raman Spectroscopy until the appropriate end point was reached. The reaction mixture was then further cooled to 20°C (± 5°C) over 3 to 5 hours (target: 4 hours) and monitored by Raman Spectroscopy until the appropriate end point is reached. The solids were filtered and washed with 3 X 2 volumes of water and dried at 45°C (± 10°C). See Table 1 and Figure 2: PXRD spectrum of Form 1 and Figure 4: PXRD of crisaborole drug product placebo lot overlaid with Form 1.

### 3. Method for Producing Form 2

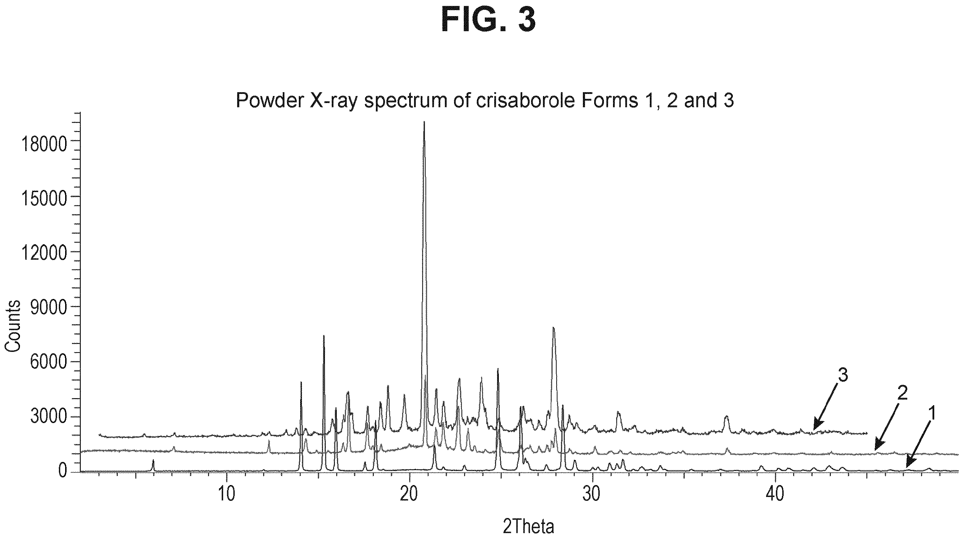

Crisaborole was dissolved in acetonitrile at 70C and approximately 2 volumes of water were added to the solution, affording immediate precipitation of Form 2. See Table 2 and Figure 3: PXRD spectrum of Forms 1, 2 and 3.

### 4. Method for Producing Form 3

Polymorphic Form 3 was typically only found during polymorph screening utilizing fast evaporation studies from solvents such as ethyl acetate, methyl ethyl ketone, and methyl tert-butyl ether. Approximately 1 g of Crisaborole was dissolved in acetonitrile at 75°C, followed by filtration. Water was added to the filtrate as an anti-solvent at 75°C. The resulting mixture was a clear solution, and it was seeded with approximately 2 mg of Form III seed crystal. The mixtures were then cooled to ambient temperature at 20 °C/h and stirred overnight. The solids were isolated by filtration and washed with approximately 2 mL of water. The solids were dried under vacuum at ambient temperature for 20 hours, which was then analyzed by XRPD to confirm the formation of Form III. See Figure 2: PXRD spectrum of Forms 1, 2 and 3.

### Solvent Selection

One of the key aspects encountered in reformulating crisaborole was that the active agent be fully dissolved within its solvent. A selection criterion of greater than 10% w/w crisaborole solubility was used to select solvents capable of carrying at least 1% crisaborole in a final drug product formulation, assuming the drug product contained 10% solvent. The solubility of crisaborole in ten solvents was evaluated to identify those with crisaborole solubility greater than 10% w/w by visual assessment. The equilibrium solubity of the best solvents was subsequently verified by HPLC. See **Table 5.**

**Table 5: Solubility Studies of Crisaborole Form II**

| Solvent | Visual Solubity^{a} (% w/w) | Solubity by HPLC^{b} (mg/mL) |
|---|---|---|
| Transcutol® P, NF | 28.6% | 21.2 |
| Hexylene Glycol, NF | 25.9% | 356 |
| Propylene Glycol, USP | 24.0% | 274 |
| Polyethylene Glycol (PEG) 400, USP | 15.0% | 164 |
| Propylene Carbonate, NF | 9.9% | 96 |
| Diisopropyl Adipate | 7.7% | -- |
| Oleyl Alcohol, NF | 2.3% | 33 |
| Ethylhexyl Hydroxystearate | 1.6% | 21 |
| Isopropyl Myristate, NF | 0.6% | -- |
| Oleic Acid, NF | <0.1 % | -- |

| | | |
|---|---|---|
| --Not tested a. Crisaborole polymorph Form 2 b. Transcutol P is equivalent to diethylene glycol monoethyl ether | | |

The solvents that satisfied the greater than 10% w/w solubility requirement were also evaluated for crisaborole compatibility by assessing degradant formation in crisaborole solutions at 80% saturation after storage at 50° C and 40 ° C/75% RH for seven days.

Crisaborole solutions of three of the seven evaluated solvents (propylene glycol, propylene carbonate and PEG400) exhibited two main degradants, including Impurity 1. Of these three, the propylene glycol solutions had the lowest overall degradation and the highest crisaborole solubility. Crisaborole solutions of the four remaining solvents all exhibited an additional degradant and, therefore, were excluded from development. (See **Table 6)**

**Table 6: Crisaborole Compatibility with Various Solvents**

| | | **Degradant (% Peak Area)** | | | |
|---|---|---|---|---|---|
| **Solvent** | **Condition** | Impurity 2 | Impurity 1 | Impurity 3 | Total |
| Propylene Glycol, USP | To | - | - | - | - |
| | 7 day, 40°C/75% RH | 0.0 | 0.0 | - | 0.15 |
| | 7 day, 50°C | 0.1 | 0.1 | - | 0.26 |
| Propylene Carbonate, NF | To | - | - | - | - |
| | 7 day, 40°C/75% RH | - | 1.3 | - | 1.37 |
| | 7 day, 50°C | 0.0 | 2.0 | - | 2.08 |
| PEG 400, USP | To | 0.2 | - | - | 0.27 |
| | 7 day, 40°C/75% RH | 0.7 | 0.0 | - | 0.82 |
| | 7 day, 50°C | 0.9 | 0.1 | - | 1.09 |
| Hexylene Glycol, NF | To | | - | - | - |
| | 7 day, 40°C/75% RH | 0.1 | 0.0 | 0.1 | 0.30 |
| | 7 day, 50°C | 0.2 | 0.1 | 0.3 | 0.74 |
| Transcutol P, NF^{a} | To | 0.1 | - | - | 0.12 |
| | 7 day, 40°C/75% RH | 0.2 | - | - | 0.22 |
| | 7 day, 50°C | 0.2 | 0.0 | 0.1 | 0.47 |
| Oleyl Alcohol, NF | To | 0.4 | - | 0.0 | 0.53 |
| | 7 day, 40°C/75% RH | 5.9 | - | 0.1 | 6.02 |
| | 7 day, 50°C | 9.1 | 0.10 | 0.2 | 9.48 |
| Ethylhexyl | To | 0.1 | - | 7.8 | 7.97 |
| | 7 day, 40°C/75% RH | 0.2 | - | 11. | 11.78 |
| | 7 day, 50°C | 0.4 | - | 13. | 13.81 |

| | | | | | |
|---|---|---|---|---|---|
| RRT, relative retention time; -, not detected. a Transcutol P is equivalent to diethylene glycol monoethyl ether. | | | | | |

### Emulsifier Selection

To form an ointment containing propylene glycol phase dispersed within a petrolatum base, studies were conducted to identify an emulsifier with an appropariate hydrophilic-lipophilic balance (HLB) value, acceptable crisaborole compatibility, and demonstrated emulsifying ability. An emulsifier with an HLB value of 4-6 was desired for a water-in-oil type emulsion, and four potential emulsifiers were evaluated for crisaborole compatibility: mono- and di-glycerides (MDG), sorbitan sesquioleate, sorbitan monooleate, and glyceryl monooleate with HLB values of 3.8, 3.7, 4.3, and 3.8 respectively.

Crisaborole was dissolved in propylene glycol to which each emulsifier was added, forming either a solution or partial suspension. The levels of each were approximately equivalent to levels in an ointment formulation containg 10% propylene glycol, 2% crisaborole and 5% emulsifier. The samples were stored at 50° C for 7 days before they were analyzed for degradation of crisaborole. The compatibility results are summarized in Table 7.

**Table 7: Crisaborole Compatibility with Various Emulsifiers**

| **Emulsifier** | **Condition** | **Degradant (% Peak Area)** | |
|---|---|---|---|
| | | Impurity X | Impurity 1 |
| Mono- and Di-glycerides ^{a} | 7 day, 50°C | | 1.2 |
| Sorbitan Sesquioleate, NF | 7 day, 50°C | | 13.2 |
| Sorbitan Monooleate, NF | 7 day, 50°C | 0.45 | 8.4 |
| Glyceryl Monooleate, NF | 7 day, 50°C | .15 | 18.9 |

| | | | |
|---|---|---|---|
| -, not detected. a Identified as Glycerol monostearate, purified. | | | |

The MDG grade initially evaluated for crisaborole compatibility was non-compendial and identified as glycerol monostearate, purified. Testing demonstrated, however, that the material conformed more closely to the mono- and di-glycerides, NF compendial specification than to the glyceryl monostearate, NF (GMS) compendial specifications. It is, therefore described herein, as MDG. In an effort to improve the formulation, the non-compendial MDG was replaced with a compendial grade of glyceryl monostearate, NF (Sasol Imwitor® 491). Sasol Imwitor® 491, however, exhibited poor emulsifying properties, likely due to different proportions of mono-glyceride and di-gyceride that is inherent between MDG and GMS.

A variety of compendial and non-compendial MDG and GMS grades were subsequently evaluated for crisaborole compatibility. For the emulsifier evaluation:
(a) a solution of the ointment active phase was prepared: 2 g crisaborole, 8.91 g propylene glycol, 0.09 g water, and 0.1 g boric acid;
(b) 1 gram of active phase was mixed with 0.6 gram of emulsifier, and
(c) the samples were stored at 50°C for 7 days.
The crisaborole compatibility results are presented in Table 8.

**Table 8: Crisaborole Compatibility with Potential Emulsifier Grades**

| | **Compendial Mono-glyceride** | | | **Degradant (% Peak)** | |
|---|---|---|---|---|---|
| **Source (Material)** | **Grade** | **Status** | **Content (%)** | Impurity | Impurity 1 |
| Control A^{a} | **-** | - | - | 0. | 0. |
| Gattefosse (Geleol ™) | MDG | NF | 5 | 0. | 0. |
| Alfa Aesar (Glycerol | MDG | none | 4 | 0. | 0. |
| Sasol (Imwitor® 491) | GMS | NF | 9 | 0. | 0. |
| Sasol (Imwitor® 900K) | MDG | NF | 4 | 0. | 1. |
| Caravan (BFP® 74K) | MDG | none | 4 | 0. | 1. |
| Caravan (BFP® 74E) | MDG | none | 4 | 0. | 3. |
| Abitec (Capmul® MCM) | MDG | NF | 5 | 0. | 2. |
| Control B^{a} | - | - | - | 0. | 0. |
| Cognis (Cutina® GMS V) | MDG | NF | 5 | 0. | 0. |

| | | | | | |
|---|---|---|---|---|---|
| GMS, glyceryl monostearate; MDG, mono- and di-glycerides; RRT, relative retention time; -, not applicable. a Control A was conducted concurrently with all emulsifiers except Cutina, which was conducted concurrently with Control B. | | | | | |

Multiple sources of mono- and di-glycerides exhibited acceptable compatibility with crisaborole under these stability conditions. Exemplary sources included Gattefosse (Geleol™), Alfa Aesar (Glycerol monostearate), Sasol (Imwitor® 491), Sasol (Imwitor® 900K), Caravan (BFP® 74K), Caravan (BFP® 74E), Abitec (Capmul® MCM NF), Cognis (Cutina® GMS V PH). Mono- and di-glycerides, NF was selected to be the emulsifier for Crisaborole Topical Ointment, 2% on the basis of compendial status, HLB value, chemical compatibility, and established emulsifying capability.

### Stabilizer Selection

Early prototype formulations demonstrated improved stability, particularly with regard to protodeboronation, when approximately 1% of the propylene glycol solvent was replaced with water. Further improvement in stability was found when a small percentage of boric acid was added to the formulation. The Crisaborole Ointment Z6 formulation, therefore, included small amounts of both water and boric acid. However, the mechanism by which water and boric acid reduced the rate of protodeboronation was not well understood.

During scale-up procedures, however, the presence of water in propylene glycol reduced the solubility of crisaborole Form I (the commercial polymorphic Form), presenting potential formulation challenges. Furthermore, the water and boric acid combination did not adequately stabilize crisaborole and an alternative stabilizer was therefore pursued.

Edetate calcium disodium (EDTA) was evaluated as a stabilizer at various levels within drug product formulations utilizing the same excipients used in Crisaborole Topical Ointment, 2%. The drug product formulations were evaluated in multiple candidate tubes, including 60-g laminate tubes equivalent to the commercial primary container closure system (with the exception of not having an orifice seal), at both 25°C/60% RH and 40°C/75% RH.

The evaluated formulations differed from Crisaborole Topical Ointment, 2% only in the stabilizers used: 0 ppm EDTA, **Z10,** 24 ppm EDTA (Ointment **Z7**), 90 ppm EDTA, 450 ppm EDTA, and 0.09% water with 0.10% boric acid (equivalent to Ointment **Z6,** but utilizing MDG rather than GMS). Results from the evaluation in the 60-g laminate tubes are presented in **Tables 5** and **6.** After six months, the formulation without stabilizer exhibited the greatest total crisaborole degradation and lowest assay at the 40°C/75% RH condition, followed by the Ointment **Z6** formulation (water and boric acid as stabilizer). The greatest stabilizing effect was observed at the 24 ppm EDTA and 90 ppm EDTA levels. The 24 ppm EDTA formulation (Ointment **Z7**) was subsequently utilized in clinical studies

### EXAMPLES

### EXAMPLE 1

### Manufacture of Topical Pharmaceutical Formulations of the invention

### a) Topical pharmaceutical formulation with an active agent

### Step 1: Preparation of Oil Phase

In a primary compounding vessel, white petrolatum, paraffin wax and mono and diglycerides were added with continuous propeller mixing while being heated to 70-80 °C. The temperature of this mixture was maintained at 70-80°C with the mixture appearing visually melted and uniform. With propeller stirring, butylated hydroxytoluene was added and mixed to dissolve while maintaining the temperature to 70-80°C. While being stirred, the mixture was cooled down to 40-46 °C and maintained until addition of drug solution phase.

### Step 2: Preparation of Drug Solution Phase

In a secondary compounding vessel, edetate calcium disodium was added to propylene glycol with continuous propeller mixing while being heated to 40-46°C. The temperature of this mixture was maintained at 40-46°C with the mixture appearing visually dissolved and uniform. With continuous mixing, crisaborole was added to dissolve while maintaining the temperature at 40-46°C.

### Step 3: Emulsification

The drug solution phase was filtered through 80 mesh filter and added to the oil phase. It was then homogenized for 10 minutes while maintaining the temperature at 40-46°C. The final composition was cooled to 25°C with propeller mixing until a homogenous ointment was obtained.

All components were compendial. The 2% ointment formulation, **Z,** was produced according to the procedure above and had the following components:

| **Component** | **Z % w/w** |
|---|---|
| Crisaborole | 2.000 |
| Propylene Glycol | 9.000 |
| Butylated Hydroxytoluene | 0.100 |
| Mono- and Di- glycerides | 7.000 |
| Paraffin Wax | 5.000 |
| White Petrolatum | 76.8965 |
| Edetate Calcium Disodium | 0.0035 |

### b) Topical pharmaceutical formulation without an active agent

### Step 1: Preparation of Oil Phase

In a primary compounding vessel, white petrolatum, paraffin wax and mono and diglycerides were added with continuous propeller mixing while being heated to 70-80 °C. The temperature of this mixture was maintained at 70-80 °C with the mixture appearing visually melted and uniform. With propeller stirring, butylated hydroxytoluene was added and mixed to dissolve while maintaining the temperature to 70-80 °C. While being stirred, the mixture was cooled down to 40-46 °C and maintained until addition of drug solution phase.

### Step 2: Preparation of Solvent Phase

In a secondary compounding vessel, edetate calcium disodium was added to propylene glycol with continuous propeller mixing while being heated to 40-46 °C. The temperature of this mixture was maintained at 40-46 °C with the mixture appearing visually dissolved and uniform.

### Step 3: Emulsification

The solvent phase was filtered through 80 mesh filter and added to the oil phase. It was then homogenized for 10 minutes while maintaining the temperature at 40-46°C. The final composition was cooled to 25°C with propeller mixing until a homogenous ointment was obtained.

All components were compendial. The 2% ointment formulation, Y, was produced according to the procedure above and had the following components:

| **Component** | **Y% w/w** |
|---|---|
| Propylene Glycol | 9.000 |
| Butylated Hydroxytoluene | 0.100 |
| Mono- and Di- glycerides | 7.000 |
| Paraffin Wax | 5.000 |
| White Petrolatum | 78.8965 |
| Edetate Calcium Disodium | 0.0035 |

### EXAMPLE 2

### Flux tests for 2% and 5% crisaborole creams-

In vitro penetration of crisaborole in cream formulations through human ex vivo cadaver skin was measured. The composition of the 2% crisaborole cream formulation, **Z1,** and the 5% crisaborole cream formulation, **Z2,** are provided below. Both **Z1** and **Z2** are disclosed in U.S. Pat. App. No. 12/399,015 (U.S. Pat. Pub. No. US2009/0291917) and PCT Pat. App. No. PCT/US09/036250 (PCT Pat. Pub. No. WO2009/111676).

| **Component** | **Z1 % w/w** | **Z2 % w/w** |
|---|---|---|
| Crisaborole | 2.0 | 5.0 |
| Methylparaben | 0.15 | 0.15 |
| Propylparaben | 0.03 | 0.03 |
| Glyceryl Monostearate SE | 8.0 | 8.0 |
| Butylated Hydroxytoluene | 0.02 | 0.02 |
| Edetate Disodium | 0.05 | 0.05 |
| Pemulen TR-2 | 0.25 | 0.25 |
| Carbopol Ultrez 10 | 0.20 | 0.20 |
| 25% Trolamine | 0.84 | 0.84 |
| Propylene Glycol | 5.0 | 5.0 |
| Octyldodecanol | 10.0 | 10.0 |
| Oleyl Alcohol | 10.0 | 10.0 |
| Benzyl Alcohol | 2.0 | 2.0 |
| Diisopropyl Adipate | 10.0 | 10.0 |
| Purified Water | QS 100 | QS 100 |

### 25% Trolamine Solution

| **Component** | **% w/w** |
|---|---|
| Trolamine | 25.0 |
| Purified Water | 75.0 |

Testing was conducted according to the following protocol.

### Study Skin Preparation

Percutaneous penetration was measured using the *in vitro* cadaver skin finite dose technique. Human, *ex vivo,* trunk skin, within 1 year of collection without obvious signs of skin disease was used in this study. It was dermatomed, prepared for cryopreservation, sealed in a water impermeable plastic bag, and stored at ≤ -70°C until the day of the experiment. Prior to use it was thawed in ∼37°C water, then rinsed in water to remove any adherent blood or other material from the surface.

Skin from a single donor was cut into multiple smaller sections large enough to fit on static 1.0 cm² Franz diffusion cells. The dermal chamber was filled to capacity with a reservoir solution of phosphate-buffered isotonic saline (PBS), pH 7.4 ± 0.1, and the epidermal cell (chimney) left open to ambient laboratory conditions. All cells were mounted in a diffusion apparatus in which the dermal bathing solution was stirred magnetically at approximately 600 RPM and the skin surface temperature maintained at 32.0 ± 1.0 °C.

To assure the integrity of each skin section, its permeability to tritiated water was determined before application of the test products. Franz TJ et al., Abst. J Invest Dermatol 1990, 94:525. Following a brief (0.5-1 hour) equilibrium period, ³H₂O (NEN, Boston, MA, sp. Act. ∼ 0.5 µCi/mL) was layered across the top of the skin by dropper so that the entire exposed surface was covered (approximately 250 - 500 µL). After 5 minutes the ³H₂O aqueous layer was removed. At 30 minutes the receptor solution was collected and analyzed for radioactive content by liquid scintillation counting. Skin specimens in which absorption of ³H₂O was less than 1.56 µL-equ/cm² were considered acceptable.

Following the water test the chambers were arranged in formulation groups so that there was an even raked distribution of the chambers with associated water penetration within each group for each formulation.

### Dosing and Sample Collection

Just prior to dosing, a pre-dose sample was taken and the reservoir solution was replaced with a fresh solution of 0.1 × PBS with 0.1 % Volpo. The chimney was removed from the Franz Cell to allow full access to the epidermal surface of the skin. All formulations were then applied to the skin sections using a positive displacement pipette set to deliver 5 µL formulation/cm². The dose was spread across the surface with the Teflon tip of the pipette. Five to ten minutes after application the chimney portion of the Franz Cell was replaced. Care was taken to keep containers of dosing solution capped when not in use and to leave them open as little as possible during dosing in order to minimize evaporation.

At pre-selected times after dosing, (4, 8, 12, 24, and 48 hours) the reservoir solution was removed in its entirety, replaced with fresh reservoir solution, and a predetermined volume aliquot saved for subsequent analysis. All samples were collected in 2 mL Boil-Proof Microtubes (Axygen Scientific MCT-200-C).

Spare cells were available which were not dosed but used to evaluate for the appearance of substances diffusing out of the skin that might interfere with the analytic method.

After the last sample was collected, the surfaces were washed twice (0.5 mL volume each) with acetonitrile to collect un-absorbed formulation from the surface of the skin. Following the wash, the skin was tape stripped (Transpore® Tape, 3M) no more than 10 times to remove the stratum corneum. The tape strips were extracted overnight in acetonitrile. The skin was then removed from the chamber, split into epidermis and dermis. Each was extracted overnight in acetonitrile. In addition, at the end of the study, the chamber parts (dosing chimney and reservoir chamber) were rinsed separately with acetonitrile and the samples retained for analysis.

### Results:

Total percutaneous penetration through human cadaver skin over 48 hours for Z1 and Z2 are provided below:

**Mean Cumulative Amounts (µg/cm²) of Crisaborole through Human Cadaver Skin over 48 Hours (Creams)**

| | Z1 | Z2 |
|---|---|---|
| Mean ± SD N=3 donors, 3 replicates per donor. | 1.13 ±0.67 µg/cm² | 4.57 ± 3.51 µg/cm² |

### EXAMPLE 3

### Flux tests for 2% crisaborole ointment Z3 and 5% crisaborole ointment Z4-

In vitro penetration of crisaborole in two ointment formulations through human ex vivo cadaver skin was measured. The composition of the 2% crisaborole ointment formulation, Z3, and the 5% crisaborole ointment formulation, Z4, are provided below. Both Z3 and Z4 are disclosed in U.S. Pat. App. No. 12/399,015 (U.S. Pat. Pub. No. US2009/0291917) and PCT Pat. App. No. PCT/US09/036250 (PCT Pat. Pub. No. WO2009/111676).

| **Component** | **Z3 % w/w** | **Z4 % w/w** |
|---|---|---|
| Crisaborole | 2.0 | 5.0 |
| Ethylhexyl Hydroxystearate | 10.0 | 10.0 |
| Oleyl Alcohol | 10.0 | 10.0 |
| White Petrolatum | 78.0 | 75.0 |

Testing was conducted according to the protocol of Example 2.

### Results:

Total percutaneous penetration through human cadaver skin over 48 hours for **Z3** and **Z4** are provided below:

**Mean Cumulative Amounts (µg/m²) of Crisaborole through Human Cadaver Skin over 48 Hours (Ointments)**

| | **Z3** | **Z4** |
|---|---|---|
| Mean ± SD N=3 donors, 3 replicates per donor. | 3.89 ± 0.87 µg/cm² | 4.43±1.81 µg/cm² |

### EXAMPLE 4

### Flux tests for 2% crisaborole cream Z1 and 2% crisaborole ointment Z -

In vitro penetration of crisaborole in 2% cream formulation **Z1** and ointment formulation **Z,** through human ex vivo cadaver skin was measured.

The composition of the 2% crisaborole cream, Z1, is described in Example 2. The composition of the 2% crisaborole ointment, Z, is described in Example 1.

### Skin Samples & Donor Demographics

Human ex vivo cadaver skin was supplied by Allosource (6278 South Troy Circle, Centennial, CO) and stored at -80°C until use. The thickness of dermatomed skin was approximately 500 µm. Human cadaver skin without obvious signs of skin disease from three donors was used during the study.

### Test Procedure

Human ex vivo cadaver skin was stored at -80°C until the morning of the study. Skin was thawed by submerging in pre-warmed phosphate buffered saline (PBS) (37°C), and was inspected for any visible holes or damage. Skin was carefully sectioned using a scalpel to the appropriate size for placement onto the vertical diffusion cell.

Receiving media (90:10 water:propylene glycol v/v) that was pre-warmed to 37°C, and a stir bar, were added to each diffusion cell and allowed to equilibrate for a minimum of 30 minutes. A section of skin was placed on top of each cell, and cells were assembled using clamps to secure the skin in place. Any air bubbles that were introduced during the assembly of the cells were removed. Skin and media were allowed to equilibrate for a minimum of thirty minutes after assembly of the cells.

A 400 µL pre-dose sample of receptor media was collected for analysis, and an equal volume was replaced with pre-warmed fresh media. Formulations were warmed to 31 ± 1 °C and equilibrated for approximately 1 hour prior to dosing. Immediately prior to dosing, formulations were briefly mixed using the pipette tip. At one minute intervals, each cell was dosed once with approximately 5 µL/cm² of respective formulation using a positive displacement pipette. A glass rod was used to spread the formulation evenly covering the entire surface area of the skin. The sampling port was occluded with parafilm to prevent evaporation of the receptor media during the study. Each glass rod was saved and the tip extracted overnight in 400 µL extraction solution (0.1% formic acid in acetonitrile).

At 3, 6, 12, 24 and 48 hours following dose administration, a 400 µL aliquot of receiving media was removed through the sampling stem of each cell with a pipette. After removing the media, an equal volume of pre-warmed fresh receiving medium was added to replace the volume removed during sampling. Care was taken to avoid generation of any air bubbles during sampling, and any bubbles were carefully removed if necessary.

At the conclusion of the study, the cells were disassembled and the skin was carefully removed from each cell. Each skin section was washed twice with 0.5 mL of extraction solution (0.1% formic acid in acetonitrile) to collect un-absorbed formulation from the surface of the skin. The skin surface was gently cleansed with lab tissue to remove any residual liquid from the wash. Tissues were tape stripped 1-2 times with 3M Transpore tape to collect the stratum corneum. Tape strips were collected, combined, and extracted in 1 mL extraction solvent (0.1% formic acid in acetonitrile).

Following tape stripping, the skin was carefully separated into epidermis and dermis using forceps. Each section was added to a tared vial and weights collected. To each epidermis vial, homogenization solution (0.1% formic acid in water/propylene glycol [10:90 v/v]) was added at a ratio of 10x tissue weight. To each dermis vial, homogenization solution was added at a ratio of 4× tissue weight. Tissues were homogenized using a bead homogenizer (Omni BeadRuptor with 2 mL micro tubes containing 2.8 mm ceramic beads) at the following settings:
Speed: 7.45 m/sec; Cycle time: 15 sec; # of Cycles: 2; Dwell time: 1 min

### Sample Analysis

The receiving medium collected from the diffusion cell assemblies was aliquotted into 96 well plates and frozen at -20°C. Epidermis and dermis homogenates and tape strip extracts of stratum corneum prepared at the conclusion of the experiment were also frozen at -20°C.

### Results

The receiving medium collected from the diffusion cell assemblies was aliquotted into 96 well plates and frozen at -20°C.

**Mean Cumulative Amounts (µglcm²) of Crisaborole penetrated through human cadaver skin into the receiving medium over 48 Hours**

| | **Z** | **Z1** |
|---|---|---|
| Mean ± SD N=3 donors, 2 replicates per donor. | 35.04 ± 10.81 µg/cm² | 20.60±10.18 µg/cm² |

The penetration of **Z** into and through human skin is greater than the penetration of **Z1.**

### EXAMPLE 5

### Flux tests for 2% crisaborole ointment Z3 with oleyl alcohol and 2% crisaborole ointment Z6 with propylene glycol-

In vitro penetration of crisaborole in two ointment formulations through human ex vivo cadaver skin was measured. The composition of the 2% crisaborole ointment formulation with oleyl alcohol, **Z3,** and the 2% crisaborole ointment formulation with propylene glycol, **Z6,** are provided below.

| **Component** | **Z3 % w/w** |
|---|---|
| Crisaborole | 2.0 |
| Ethylhexyl Hydroxystearate | 10.0 |
| Oleyl Alcohol | 10.0 |
| White Petrolatum | 78.0 |

| **Component** | **Z6 % w/w** |
|---|---|
| Crisaborole | 2.00 |
| Propylene Glycol | 8.91 |
| Boric Acid | 0.10 |
| Purified Water | 0.09 |
| Butylated Hydroxytoluene | 0.10 |
| Mono- and Di- Glycerides, 40-55% Monoglycerides | 7.00 |
| Paraffin wax | 5.00 |
| White Petrolatum | 76.80 |

### Skin Samples & Donor Demographics:

Human ex vivo cadaver skin without obvious signs of skin disease was used in the study. It was stored at ∼-70°C until use. It was dermatomed, prepared for cryopreservation, sealed in a water impermeable plastic bag, and stored at ∼ -70°C until the day of the experiment.

### Test Procedure:

Human ex vivo cadaver skin was stored at ∼-70°C until the morning of the study. Skin was thawed by submerging in pre-warmed water (37°C), and was inspected for any visible holes or damage. Skin from a single donor was cut into multiple smaller sections large enough to fit on nominal 1.0 cm² static Franz diffusion cells.

Skin from a single donor was cut into multiple smaller sections large enough to fit on nominal 1.0 cm² static Franz diffusion cells. The dermal chamber was filled to capacity with a reservoir solution of phosphate-buffered isotonic saline (PBS), pH 7.4 ± 0.1, and the epidermal cell (chimney) left open to ambient laboratory conditions. All cells were mounted in a diffusion apparatus in which the dermal bathing solution was stirred magnetically at approximately 600 RPM and the skin surface temperature maintained at 32.0 ± 1.0 °C.

A water integrity test was determined before application of the test products. Following a brief (0.5-1 hour) equilibrium period, ³H₂O (Perkin Elmer, sp. Act. ∼ 0.5 µCi/mL) was layered across the top of the skin so that the entire exposed surface was covered (approximately 250 - 500 µL). After 5 minutes the ³H₂O aqueous layer was removed. At 30 minutes the receptor solution was collected and analyzed for radioactive content by liquid scintillation counting. Skin specimens in which absorption of ³H₂O was less than 1.56 µL-equ/cm² were considered acceptable.

Just prior to dosing, the reservoir solution was replaced with a fresh solution of distilled deionized water (ddH₂O) with 80 µg/mL gentamicin. The chimney was removed from the Franz Cell to allow full access to the epidermal surface of the skin. The test formulations were warmed slightly at a controlled temperature of 30 ± 2 °C and equilibrated at the maintained temperature for approximately 2 hours prior to dosing.

The product was applied to five replicate sections of the same donor skin. Dosing was performed using a positive displacement pipette set to deliver 5µL formulation/cm², or by weight (5 mg/cm²) if not of sufficient viscosity to pipette.

### Results:

Total percutaneous penetration through human cadaver skin over 48 hours for **Z3** and **Z6** are provided below:

**Mean Cumulative Amounts (µg/cm²) of Crisaborole penetrated through human cadaver skin into the receiving medium over 48 Hours**

| | **Z3** | **Z6** |
|---|---|---|
| Mean ± SD N=2 donors, 5 replicates per donor. | 68.047± 8.15 µg/cm² | 82.212± 5.18 µg/cm² |

Flux of crisaborole through the skin was surprisingly greater with **Z6** than with **Z3**.

### EXAMPLE 6

### Physical Stability Centrifugation Stress Tests

Pharmaceutical formulation Z5 contained the following ingredients:

| **Component** | **Z5 % w/w** |
|---|---|
| Crisaborole | 2.00 |
| Propylene Glycol | 8.91 |
| Boric Acid | 0.10 |
| Purified Water | 0.09 |
| Butylated Hydroxytoluene | 0.10 |
| Mono- and Di- Glycerides, 90% Monoglycerides | 7.00 |
| Paraffin wax | 5.00 |
| White Petrolatum | 76.80 |

and was prepared as described in Example 1 above, but with boric acid and water being added in Step 2A instead of EDTA.

Pharmaceutical formulation **Z6** contained the following ingredients:

| **Component** | **Z6 % w/w** |
|---|---|
| Crisaborole | 2.00 |
| Propylene Glycol | 8.91 |
| Boric Acid | 0.10 |
| Purified Water | 0.09 |
| Butylated Hydroxytoluene | 0.10 |
| Mono- and Di- Glycerides, 40-55% Monoglycerides | 7.00 |
| Paraffin wax | 5.00 |
| White Petrolatum | 76.80 |

and was prepared as described for Z5 above.

The physical stability of **Z5** and **Z6** were tested by centrifugation. Samples of each ointment were placed in 15 mL low density polyethylene centrifuge tubes. The sample size was approximately 10 mL per centrifuge tube. Samples were equilibrated for about one hour in a 25°C/60% RH oven before centrifugation. The tubes were placed in a Beckman Coulter Allegra 6R Series Centrifuge and spun at 2890 rpm. After spinning for 1.5 hours, the percentage of internal volume separation was measured.

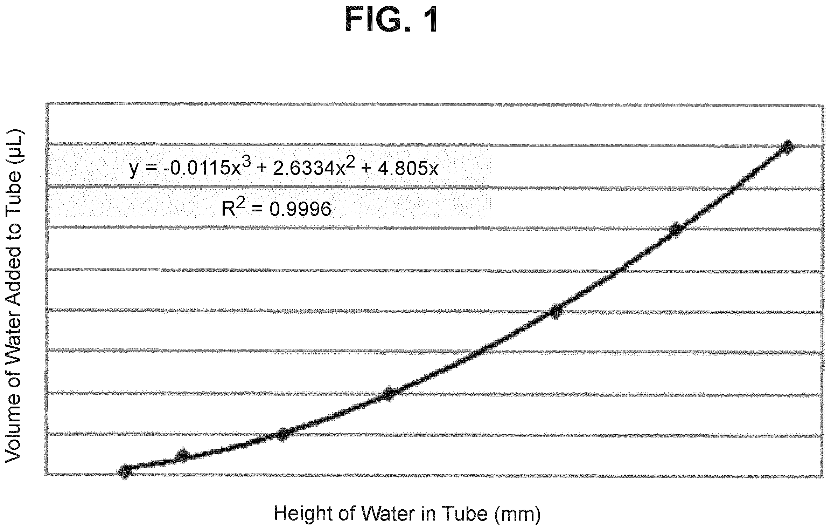

The volume of material that physically separated was determined by measuring the height of the separated phase and calculating the volume using the equation provided in Figure 1. The percent of the total sample that separated was then calculated by dividing the volume of the separated phase by the total ointment volume.

**Z6** measured 0% internal volume separation while **Z5** measured 1.4% internal volume separation. The ointments were next spun for an additional 1.5 hours (3.0 hours total), and the percentage of internal volume separation was measured again. **Z6** measured 0% internal volume separation while **Z5** measured 1.5% internal volume separation.

Ointment formulations utilizing mono- and di- glycerides (MDG) in which the monoglyceride percentage is between 40-55% possess greater physical stability over ointment formulations utilizing mono- and di- glycerides (MDG) in which the monoglyceride percentage is at least 90%.

### EXAMPLE 7

### Degradant Reduction Tests:

Impurities in stability batches have been observed and monitored, including Impurity 1, which is believed to be a stability protodeboronation product of crisaborole.

### Impurity 1:

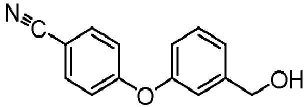

Impurity 1 was characterized with nuclear magnetic resonance spectroscopy (NMR), mass spectrometry (MS) and the retention time of high-performance chromatography (HPLC) was confirmed against the standard. The H and C position assignments for Impurity 1 based on the NMR data are listed in **Table 2.**

**Table 2: ¹H NMR and ¹³C NMR Assignments for Impurity1**

| **Position** ^{a} | **δ¹H (ppm)** | **Multiplicity J_{HH} (Hz)** | **δ¹³C (ppm)** |
|---|---|---|---|
| 1 | - | - | 105.02 |
| 2 | 7.84 | d, | 134.63 |
| 3 | 7.08 | m | 118.04 |
| 4 | - | - | 161.16 |
| 5 | 7.08 | m | 118.04 |
| 6 | 7.84 | d, | 134.63 |
| 7 | 7.00 | dd, 7.9, 2.4 | 118.37 |
| 8 | 7.41 | apparent t, 7.8 | 130.08 |
| 9 | 7.21 | d, | 122.96 |
| 10 | - | - | 145.57 |
| 11 | 7.08 | m | 117.79 |
| 12 | - | - | 154.44 |
| 13 | 4.52 | d, | 69.26 |
| 14 | 5.28 | t, 5.8 | - |
| 15 | - | - | 118.73 |

| | | | |
|---|---|---|---|
| d, doublet; dd, doublet of a doublet; m, multiplet; t, triplet. a Position assignment was confirmed by extended NMR (gCOSY, NOESY 1D, gHSQC and gHMBC). | | | |

The proposed acceptance criterion to the FDA for Impurity 1 is not more than 2.0% crisaborole label strength, based upon stability data. Impurity 1 levels were observed to increase over time, up to 1.2% label strength and 1.4% label strength for the primary and supporting stability lots under long-term (25° C, 60% relative humidity) and accelerated (40° C, 75% relative humidity) storage conditions, respectively.

Accordingly, minimizing the amount of degradation of crisaborole is a goal of the formulation.

Pharmaceutical formulations with different types and amounts of stabilizers (**Z**, **Z6, Z7, Z8, Z9,** and **Z10**) were tested for their ability to reduce the amount of **Impurity 1**.

The compositions of **Z** and **Z6** are described herein. The 2% ointment formulations **Z7, Z8, Z9,** and **Z10,** have the following components and were produced according to the methods described herein:

| **Component** | **Z7 % w/w** | **Z8 % w/w** | **Z9 % w/w** | **Z10 % w/w** |
|---|---|---|---|---|
| Crisaborole | 2.000 | 2.000 | 2.000 | 2.000 |
| Propylene Glycol | 9.000 | 9.000 | 9.000 | 9.000 |
| Butylated Hydroxytoluene | 0.100 | 0.100 | 0.100 | 0.100 |
| Mono- and Di- glycerides | 7.000 | 7.000 | 7.000 | 7.000 |
| Paraffin Wax | 5.000 | 5.000 | 5.000 | 5.000 |
| White Petrolatum | 76.8976 | 76.9810 | 76.855 | 76.900 |
| Edetate Calcium Disodium | 0.0024 | 0.0090 | 0.0450 | 0.0000 |

In summary, these formulations contain the following types and amounts of stabilizers.

| | | | |
|---|---|---|---|
| **Z6:** | Stabilizer: Boric Acid; Water | Amt: | 0.1% w/w; 0.09% w/w |
| **Z7:** | Stabilizer: Edetate Calcium Disodium | Amt: | 0.0024% w/w |
| **Z8:** | Stabilizer: Edetate Calcium Disodium | Amt: | 0.0090% w/w |
| **Z9:** | Stabilizer: Edetate Calcium Disodium | Amt: | 0.0450% w/w |
| **Z10:** | Stabilizer: None | | |
| **Z:** | Stabilizer: Edetate Calcium Disodium | Amt: | 0.0035% w/w |

### Analytical method for analysis of Impurity 1:

Crisaborole containing ointments are assayed using a reverse phase HPLC method using a BDS Hypersil C18 column (150x4.6mm, 5 micron) and a 1.0 mL/min flow rate, a 10 µL injection volume, with UV detection at 254 nm. The mobile phase (gradient) are described below:

| | |
|---|---|
| Mobile Phase A: | 0.1 %phosphoric acid solution/acetonitrile 95%/5% (v/v) |
| Mobile Phase B: | 0.1 %phosphoric acid solution/acetonitrile 5%/95% (v/v) |

The topical pharmaceutical formulations were subjected to chemical stability testing with the following results in Table 5:

**Table 5: Effect of Stabilizer on Impurity 1 formation in ointments under various conditions Impurity 1 (%)**

| **Time (Months)** | **Z6** | **Z7** | **Z8** | **Z9** | **Z10** |
|---|---|---|---|---|---|
| 0 | 0.07 | 0.12 | | | ND |

| 40 °C/75% RH | | | | | |
|---|---|---|---|---|---|
| 1 | 0.31 | 0.16 | 0.18 | 0.27 | 0.35 |
| 3 | 0.88 | 0.53 | 0.64 | 0.88 | 1.13 |
| 6 | 1.73 | 1.07 | 1.25 | 1.63 | 2.08 |

| 25 °C/60% RH | | | | | |
|---|---|---|---|---|---|
| 1 | 0.07 | 0.03 | 0.04 | 0.06 | 0.08 |
| 3 | 0.14 | 0.09 | 0.10 | 0.15 | 0.17 |
| 6 | 0.25 | 0.18 | 0.20 | 0.29 | 0.30 |

| | | | | | |
|---|---|---|---|---|---|
| ND, not detected; RH, relative humidity. | | | | | |

**Table 5** demonstrates that among these recited formulations, the formulation without stabilizer, **Z10,** exhibited the highest Impurity 1 levels. The greatest stabilizing effect was observed at the 24 ppm EDTA (**Z7**) and 90 ppm EDTA (**Z8**) levels.

Additional stability testing was performed on **Z6, Z7,** and **Z** and presented in **Table 6:**

**Table 6: Effect of Stabilizer on Impurity 1 formation in ointments under various conditions Impurity 1 (%)**

| **Time (Months)** | **Z6** | **Z7** | **Z** |
|---|---|---|---|
| 0 | 0.12 | 0.12 | ND |

| 40 °C/75% RH | | | |
|---|---|---|---|
| 1 | 0.69 | 0.49 | 0.21 |
| 3 | 1.6 | 1.0 | 0.62 |
| 6 | 2.5 | 1.6 | 1.2 |

| 25 °C/60% RH | | | |
|---|---|---|---|
| 1 | 0.22 | 0.19 | 0.07 |
| 3 | 0.46 | 0.35 | 0.12 |
| 6 | 0.66 | 0.54 | 0.25 |
| 9 | 0.82 | 0.74 | 0.29 |
| 12 | 1.1 | 0.89 | 0.37 |
| 18 | 1.7 | 1.2 | 0.54 |
| 24 | 2.0 | 1.6 | 0.73 |

| | | | |
|---|---|---|---|
| ND, not detected; RH, relative humidity. | | | |

These tests demonstrate that a much lower amount of Impurity 1 occurs with pharmaceutical formulation **Z** over formulations **Z6** and **Z7.**

### EXAMPLE 8

### Safety and Efficacy of Z and Y in the treatment of mild-to-moderate atopic dermatitis (AD)

The objective of the trial was to determine the safety and efficacy of **Z** applied twice daily (BID) compared to **Y** in the treatment of mild-to-moderate atopic dermatitis (AD) in children, adolescents, and adults (ages 2 years and older). The composition of **Z** is:

| **Component** | **Z % w/w** |
|---|---|
| Crisaborole | 2.000 |
| Propylene Glycol | 9.000 |
| Butylated Hydroxytoluene | 0.100 |
| Mono- and Di- glycerides | 7.000 |
| Paraffin Wax | 5.000 |
| White Petrolatum | 76.8965 |
| Edetate Calcium Disodium | 0.0035 |

The composition of **Y** is:

| **Component** | **Y % w/w** |
|---|---|
| Propylene Glycol | 9.000 |
| Butylated Hydroxytoluene | 0.100 |
| Mono- and Di- glycerides | 7.000 |
| Paraffin Wax | 5.000 |
| White Petrolatum | 78.8965 |
| Edetate Calcium Disodium | 0.0035 |

Two multi-center, double-blind, vehicle-controlled studies were conducted in the U.S. which enrolled over 750 patients each. Patients enrolled were aged 2 years and older with mild-to-moderate atopic dermatitis affecting >5% body surface area. Patients were randomized 2:1 (**Z:Y**) and treated twice daily for 28 days.

The primary efficacy endpoint was defined as the achievement of "Clear" (0) or "Almost Clear" (1) status, with 2 or greater grade improvement from baseline, at Day 29 according to the Investigator's Static Global Assessment (ISGA).

The secondary efficacy endpoint was defined as the achievement of "Clear" (0) or "Almost Clear" (1) status, irrespective of improvement from baseline, at Day 29 according to the Investigator's Static Global Assessment (ISGA).

In one trial, 503 patients received Z while 256 received **Y.** The mean age of those receiving **Z** was 12 years, with a range of 2 to 65 years. The mean age of those receiving **Y** was 12.4 years, with a range of 2 to 63 years. 39.0% of those receiving **Z** had a baseline ISGA of "Mild" (2), while 61.0% of those receiving **Z** had a baseline ISGA of "Moderate" (3). 36.3% of those receiving **Y** had a baseline ISGA of "Mild" (2), while 63.7% of those receiving **Y** had a baseline ISGA of "Moderate" (3). The mean % of Body Surface Area affected by atopic dermatitis for those receiving **Z** was 18.8%, with a range of from between 5% to 95%. The mean % of Body Surface Area affected by atopic dermatitis for those receiving **Y** was 18.6%, with a range of from between 5% to 90%.

From this trial, 32.8% of those receiving **Z** achieved the primary endpoint, while 25.4% of those receiving **Y** achieved the primary endpoint. 51.7% of those receiving **Z** achieved the secondary endpoint, while 40.6% of those receiving **Y** achieved the secondary endpoint.

In another trial, 513 patients received **Z** while 250 received **Y.** The mean age of those receiving **Z** was 12.6 years, with a range of 2 to 79 years. The mean age of those receiving **Y** was 11.8 years, with a range of 2 to 79 years. 38.4% of those receiving **Z** had a baseline ISGA of "Mild" (2), while 61.6% of those receiving **Z** had a baseline ISGA of "Moderate" (3). 40.0% of those receiving **Y** had a baseline ISGA of "Mild" (2), while 60.0% of those receiving Y had a baseline ISGA of "Moderate" (3). The mean % of Body Surface Area affected by atopic dermatitis for those receiving **Z** was 17.9%, with a range of from between 5% to 95%. The mean % of Body Surface Area affected by atopic dermatitis for those receiving **Y** was 17.7%, with a range of from between 5% to 90%.

From this trial, 31.4% of those receiving **Z** achieved the primary endpoint, while 18.0% of those receiving **Y** achieved the primary endpoint. 48.5% of those receiving **Z** achieved the secondary endpoint, while 29.7% of those receiving **Y** achieved the secondary endpoint.

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference in their entirety for all purposes.

## Claims

1. A topical pharmaceutical formulation comprising:
(a) 5-(4-cyanophenoxy)-1,3-dihydro-1-hydroxy-2,1-benzoxaborole, or a pharmaceutically acceptable salt, or a hydrate or a solvate thereof;
(b) from about 5% (w/w) to about 15% (w/w) propylene glycol; and
(c) petrolatum.

2. The topical pharmaceutical formulation of claim 1, wherein the formulation comprises no more than about 0.5% (w/w) water.

3. The topical pharmaceutical formulation of claim 2, wherein the amount of the propylene glycol is about 9% (w/w).

4. The topical pharmaceutical formulation of claim 3, further comprising a glyceride blend from about 3% (w/w) to about 10% (w/w), wherein the glyceride blend comprises a monoglyceride and a diglyceride.

5. The topical pharmaceutical formulation of claim 4, wherein the amount of the glyceride blend is about 7% (w/w) and the glyceride blend is Mono- and Di-glyceride NF.

6. The topical pharmaceutical formulation of claim 5, wherein from about 40% (w/w) to about 55% (w/w) of the glyceride blend is a monoglyceride.

7. The topical pharmaceutical formulation of a preceding claim, further comprising ethylenediaminetetraacetic acid, or a pharmaceutically acceptable salt thereof, from about 0.0001% (w/w) to about 0.01% (w/w).

8. The topical pharmaceutical formulation of claim 7, further comprising a sodium salt or a potassium salt or a calcium salt, or a mixture thereof, of ethylenediaminetetraacetic acid.

9. The topical pharmaceutical formulation of claim 7, further comprising edetate calcium disodium.

10. The topical pharmaceutical formulation of claim 7 or 9, wherein the amount of the ethylenediaminetetraacetic acid, or a pharmaceutically acceptable salt thereof, is about 0.0035% (w/w).

11. The topical pharmaceutical formulation of a preceding claim, further comprising an antioxidant from about 0.01% (w/w) to about 1% (w/w) selected from the group consisting of butylated hydroxytoluene, ascorbic acid or a pharmaceutically acceptable salt thereof, ascorbic palmitate, butylated hydroxyanisole, 2,4,5-trihydroxybutyrophenone, 4-hydroxymethyl-2,6-di-tertbutylphenol, erythorbic acid, gum guaiac, propyl gallate, thiodipropionic acid, dilauryl thiodipropionate, tert-butylhydroquinone and tocopherols such as vitamin E, and the like, including pharmaceutically acceptable salts and esters thereof, and mixtures thereof.

12. The topical pharmaceutical formulation of claim 11, wherein the antioxidant is butylated hydroxytoluene.

13. The topical pharmaceutical formulation of claim 11 or 12, wherein the amount of the antioxidant is 0.1% (w/w).

14. The topical pharmaceutical formulation of a preceding claim, further comprising a stiffening agent from about 2% (w/w) to about 8% (w/w).

15. The topical pharmaceutical formulation of claim 14, wherein the stiffening agent is selected from the group consisting of beeswax, paraffin wax, wax, and spermaceti wax.

16. The topical pharmaceutical formulation of claim 15, wherein the stiffening agent is 5% (w/w) paraffin wax NF.

17. A topical pharmaceutical formulation comprising:
a) 5-(4-cyanophenoxy)-1,3-dihydro-1-hydroxy-2,1-benzoxaborole, a pharmaceutically acceptable salt, or a hydrate or a solvate thereof;
b) from about 5% (w/w) to about 15% (w/w) propylene glycol;
c) butylated hydroxytoluene;
d) edetate calcium disodium;
e) mono- and di- glycerides;
f) paraffin wax; and
g) white petrolatum.

18. The topical pharmaceutical formulation of claim 17 comprising:
a) from about 0.1% (w/w) to about 2% (w/w) 5-(4-cyanophenoxy)-1,3-dihydro-1-hydroxy-2,1-benzoxaborole;
b) from about 5% (w/w) to about 15% (w/w) propylene glycol USP;
c) 0.1% (w/w) butylated hydroxytoluene;
d) 0.0035% (w/w) edetate calcium disodium;
e) 7% (w/w) mono- and di- glycerides NF;
f) 5% (w/w) paraffin wax; and
g) 76.8965% (w/w) white petrolatum.

19. The topical pharmaceutical formulation of claim 18 consisting of:
a) about 2% (w/w) 5-(4-cyanophenoxy)-1,3-dihydro-1-hydroxy-2,1-benzoxaborole, or a pharmaceutically acceptable salt thereof;
b) about 9% (w/w) propylene glycol USP;
c) about 0.1% (w/w) butylated hydroxytoluene;
d) about 0.0035% (w/w) edetate calcium disodium;
e) about 7% (w/w) mono- and di- glycerides NF, wherein between 40% and 55% is said monoglyceride;
f) about 5% (w/w) paraffin wax; and
g) about 76.8965% (w/w) white petrolatum.

20. A method of decreasing the release of a cytokine and/or a chemokine, the method comprising contacting a cell with the topical pharmaceutical formulation of a preceding claim.

21. A method of treating an inflammatory-related condition in an animal, the method comprising administering to the animal a therapeutically effective amount of the topical pharmaceutical formulation of a preceding claim.

22. The method of claim 21, wherein the inflammatory-related condition is psoriasis.

23. The method of claim 22, wherein the inflammatory-related condition is atopic dermatitis.

24. A method of treating atopic dermatitis in a human, the method comprising administering to the human a therapeutically effective amount of the topical pharmaceutical formulation of a preceding claim.

25. The method of claim 24 further comprising administrating the pharmaceutical formulation to an affected area of the human on a twice daily basis.

26. The method of claim 25 further comprising administering the pharmaceutical formulation over a period of about 28 days.

27. The method of claims 24 further comprising a second active agent administered in combination with pharmaceutical formulation.

28. The method of claim 27 wherein the second active agent is a JAK kinase inhibitor such as Tofacitinib, JTE-052, Baricitinib, or Upadacitinib.
